# EUROPEAN PATENT APPLICATION

(11) **EP 2 329 810 A1**
(43) Date of publication of application: **08.06.2011**
(21) Application number: 08876775.1
(22) Date of filing: 04.11.2008
(51) Int. Cl.: A61K 9/00

(54) **GASTRIC RETENTION DRUG DELIVERY SYSTEM, PREPARATION METHOD AND USE THEREOF**

(30) Priority: 18.08.2008 CN 200810147445
(71) Applicant: TEAM ACADEMY OF PHARMACEUTICAL SCIENCE, Feng Tai District Beijing 100039 (CN)
(72) Inventor: JIANG, Qingwei, Beijing 100039 (CN); ZHENG, Junli, Beijing 100039 (CN); YANG, Wenbin, Beijing 100039 (CN); LIU, Quanzhi, Beijing 100039 (CN)
(74) Representative: von Kreisler Selting Werner
(86) International application number: PCT/CN2008/072917
(87) International publication number: WO 2010/020098

(57) **Abstract**

A gastroretentive drug delivery system is provided. Said system comprises a hollow vesicle and a drug-containing layer which surrounds the hollow vesicle. Said hollow vesicle preferably has a single chamber structure. The size in maximal diameter direction of said hollow vesicle is preferably 0.5-3.5 cm. The gastroretentive drug delivery system preferably contains an isolating layer and/or waterproofing layer between the hollow vesicle and the layer containing drug.

## Description

### FIELD OF INVENTION

The present invention relates to the field of gastroretentive drug release. In particular, the present invention relates to a new gastroretentive drug carrier, a new gastroretentive drug delivery system and the methods for preparing the same and uses thereof.

### BACKGROUND OF THE INVENTION

Gastroretentive formulation, an important form of a gastroretentive drug delivery system, is a special formulation that is designed according to the principles of the hydronamically balanced sustained/controlled drug delivery system (HBS) and can float on gastric juice and release drug continuously after oral administration. Since its density is less than that of gastric content, such formulation usually keeps floating and remains in the stomach for 5-6h. Thus, the release time of the drug in the stomach is extended so that the drug as much as possible could reach the absorption site, thereby the drug absorption is improved, the bioavailability is enhanced and the action time of the drug is prolonged. Other advantages of gastric floating formulations include: (1) promoting absorption of weakly acidic drugs and drugs that can be absorbed in the stomach and the upper small intestine; (2) allowing drugs that are instable in the intestine tract to be absorbed fully in stomach; (3) allow drugs to play local therapeutic effects in the stomach or duodenum; and/or (4) prolonging the time of the formulation staying in the digestive tract through retaining in the stomach, so that the drugs that could be absorbed in each segments of the gastrointestinal tract are absorbed fully, thus contributing to prepare formulations administered once a day.

The gastric floating formulation was first described in detail by Tossounian and Sheth and was called "bioefficient product". In the early 80's of the 20st century, Hofmann-La Roche Co. Ltd. first launched two floating formulations, i.e. Valrelease and Valium CR. At present, gastric floating formulations are also marketed in China, such as gentamicin gastric floating sustained release tablet (trade name: SWIBEC, Jiangxi Pharmaceutical Co., Ltd., China) and so on.

In general, ideal gastroretentive formulations are believed to have the following properties: 1) after exposure to gastric juice at body temperature, the surface of gastroretentive formulation is hydrated to form a gel barrier, and the gastroretentive formulation swells, but the original shape of the gastroretentive formulation is still maintained; 2) the composition of the formulation advantageously facilitate the formulation to retain in the stomach and maintain a floating state, that is to say, the density of the formulation is less than 1g/cm³; 3) the nature and dosage of the drug and the choice of excipients should meet the requirements for *in vivo* and *in vitro* release characteristics for the gastroretentive formulation, and the drug in the formulation can be slowly dissolved and diffused, and the formulation can retain in the stomach and release the drug for a long time, generally 5-6h.

For traditional gastric floating formulationa, the main factors that affect floating properties are as follows:
1) The selection of materials: the density of hydrophilic matrix used must be less than 1g/cm³, and the hydrophilic matrix can remain in the gastric juice for a long time. Floating promoting agents and foaming agents may be added as required to reduce the density of the formulation and increase floating force;
2) Preparation process: full-automatic powder shaping is usually used to keep the pores and space in dry powder; and
3) The density and floating-holding ability of the dry formulation.

In current patent and non-patent literatures, low-density waxy materials and highly swellable materials are usually used in the floating formulations to reduce the density of the whole formulation by increasing the volume of the formulation and producing gas, so as to prolong the residence time in the stomach.

US 4,101,650 (Zaidan Hojin Biseibutsu Kagaku Kenkyu Kai) discloses a formulation, wherein particles containing sodium bicarbonate, lactose and povidone were coated with a layer of hydroxypropyl methyl cellulose, and then coated thereon with a layer of a suspension containing pepstatin as the active ingredient and hydroxypropyl methyl cellulose, to form floating minicapsules with a diameter of 0.1-2 mm. The disadvantage of such a system is that the size of the minicapsules is much smaller than that required for the normal formulations retaining in the stomach for a long time (>10mm). Sodium bicarbonate encapsulated in the HPMC layer to produce gases could not significantly reduce the density of the particles, and thus failed to meet the critical requirements for floating formulations with respect to the size and density.

US 4,777,033 (Teijin Limited) discloses an oral sustained release pharmaceutical preparation comprising low alkyl ether of cellulose, polyacrylic acid or its pharmaceutically acceptable salt, active drugs and an effective amount of foaming agent. However, the tablets made of the composition still have the major disadvantages mentioned above, because the tablet core of the system can not retain intact in decomposition test.

US 4,844,905 (Eisai Co., Ltd.) discloses a preparation comprising a core containing a pharmaceutical effective ingredient, a middle gas-generating layer comprising sodium carbonate and an organic acid, and an outer layer containing granules made of an expendible polymer film. However, due to gastric motility, the bubbles on the surface of the granules can not remain for a long time, so the buoyancy of the granules decrease continuously, and together with too small granule volume, the preparation can not float continuously and maintain enough floating time, thereby the time remaining in the stomach without being discharged can not be ensured.

Japanese Patent No. 63014715 (Sai Liya Drug Industry Co., Ltd) discloses a sustained release composition, the composition comprising (A) highly swellable hydrophilic polymer, preferably cellulose ether or polyvinyl alcohol, (B) insoluble crosslinked povidone, and (C) a component that can generate foams upon exposure to gastric juice, preferably carbonates, especially calcium carbonate or precipitated calcium carbonate. The system does not contain a drug in which one part is released immediately and the other part is released in a controlled way, that is to say, the drug is not released at two phases. Therefore, when no drug is existed in the body soon after administration, the drug release rate of the system is relatively slower than that of the drug delivery system releasing the drug at two speeds. Another disadvantage is that only highly swellable and rapidly swellable polymer may comply with the requirements for gastric retention, however, we found that several commonly used cellulose ethers do not meet this requirement.

US 5,651,985 (Bayer Aktiengesellschaft) discloses a pharmacologically active composition, comprising a drug dispersed in povidone and an acid, and a gas-generating additive. The swelling rate of the system is relatively slow, so that it can not achieve the required size and density in 30 minutes, thereby can not achieve sustained floating. In addition, the process for preparing the composition is complicated and expensive.

WO 00/15198 (Ranbaxy Lab Ltd.) relates to a gastric floating pharmaceutical composition, comprising a drug, a gas-generating composition, a swelling agent and a thickener, and optionally a gel-forming polymer. The gas-generating agent used is carbonate or bicarbonate. The swelling agent is cross-linked povidone, cross-linked carboxymethyl cellulose and sodium carboxymethyl starch. The thickener is a carbohydrates gum that can rapidly increase viscosity.

WO 01/10419 (Ranbaxy Lab Ltd.) also relates to a gastro-retentive pharmaceutical composition comprising a drug, a sugar, a diluent and a gas-generating agent. WO 01/10405 (Ranbaxy Lab Ltd.) also relates to a pharmaceutical composition comprising a drug, an inert oil, a sugar, a diluent and a gas-generating agent.

WO 00/23045 (Sanofi-Synthelabo) discloses a pharmaceutical composition with two or three layers, the composition comprising an active pharmaceutical ingredients and an excipient modifying its release and a system that can generate carbon dioxide in a swelling polymer hydrophilic tablet. In an embodiment, the active ingredient is included in the layer in which the swelling polymer is used as an excipient to modify its release, and the second layer contains the swelling polymer and carbonate. The composition is a tablet with two or three layers.

WO 01/10417 (Galenix Development) discloses and presents a pharmaceutical composition consisting of at least a first phase comprising an active principle and one or more excipients, and a non-active second phase. Said second phase comprises at least a gas-generating system and at least a hydrophilic polymer or porous mineral compound, wherein the active phase contains at least 80% of the active ingredient. The formulation that can not only obtain a desired release rate but also ensure a high reproducibility of the release performance among batches is limited by the use of release rate control agents of less than 20%. On the other hand, it should be careful to select the release rate control agent, so that a high reproducibility of the release performance can be ensured even if the very small amount of the excipient is used. Therefore, the excipient itself might not swell quickly and highly. A non-active phase is used in the system to achieve floating disclosed in WO 01/10417, that is to say, the density is lowered by surrounding the non-active phase with carbon dioxide, but the floating stability is poor.

A gentamicin gastric floating sustained release tablet (trade name: SWIBEC) is manufactured and marketed by Jiangxi Pharmaceutical Co., Ltd. in China, in which octadecanol, acrylic resins and hydroxypropyl cellulose are used as the matrix of the tablet core to control the density of the entire tablet core between 1.1-1.2 g/cm³. The main disadvantage of this preparation is that the density of the entire tablet core is high and the pressure has a great impact on the floating capacity of the tablet core. In-vitro release test shows that floating is achieved by increasing volume induced by the swelling of HPMC after 15-30min, and the swelling capacity of the tablet core is limited, thus, the floating effect in the stomach is not stable and greatly affected by gastric environment.

Although the above existing gastroretentive drug delivery systems can achieve the purpose of floating, they have one or more of the following disadvantages: complex structure, use of a large amount of excipients, complicated process, high cost and poor stability. Furthermore, the initial density of the formulation is large and it need some time for the formulation to swell, therefore, the formulation can not float immediately at the initial stage after administration, and the floating capacity (usually expressed as the floating-holding ability) thereof will reduce with the decrease of the erosion of the tablet core.

Therefore, there is still a need in the prior art for a new gastroretentive drug delivery system that has enough floating ability in the stomach and can achieve the effect of releasing the drug in the stomach.

### SUMMARY OF THE INVENTION

Surprisingly, the present inventor found that the gastroretentive of pharmaceutical preparations can be achieved effectively by coating the hollow vesicle with a drug-containing layer.

Thus, in one aspect, the present invention provides an gas-vesicle-type gastroretentive drug carrier comprsing a hollow vesicle and optionally a waterproofing layer and/or isolating layer coated on the surface of the hollow vesicle. In the present invention, the term "gastroretentive drug carrier" refers to any structure or means that can load a drug and deliver it to the stomach to realize gastric retention. In one embodiment of the present invention, the gastroretentive drug carrier comprises a hollow vesicle and optionally a waterproofing layer and/or isolating layer coated on the surface of the hollow vesicle. In another aspect, the present invention provides a new gas-vesicle-type gastroretentive drug delivery system comprising a hollow vesicle or the gastroretentive drug carrier mentioned above and a drug-containing layer coated on the surface of the hollow vesicle. The gastroretentive drug carrier or release system of the present invention may be presented as single chamber type, that is to say, one unit of the carrier or formulation only contains one hollow vesicle. Similarly, the gastroretentive drug delivery system of the present invention may also be presented as multi-chamber type, that is to say, one unit of formulation contains two or more hollow vesicles. The overall density of the formulation can be greatly reduced to less than 1.0g/cm³ and thereby can float on the gastric juice through the hollow vesicle.

Optionally, a isolating layer and/or waterproofing layer may be included between the hollow vesicle and the drug-containing layer of the gas-vesicle-type gastroretentive drug delivery system, so as to protect the hollow vesicle from being degradated by gastric juice or provent liquid from leaking into the hollow vesicle to affect the density of the formulation and further affect the floating performance in the stomach. Additionly or alternatively, a gas-generating agent may be included in the hollow vesicle of the gas-vesicle-type gastroretentive drug delivery system. The gas-generating agent can generate gas to keep the low density of the formulation and the floating performance in the stomach after the gastric juice leaking into the hollow vesicle.

In another aspect, the present invention provides a method for preparing the gas-vesicle-type gastroretentive drug delivery system according to the present invention, comprising the following steps: (1) preparing or providing a hollow vesicle, and optionally coating the hollow vesicle with a waterproofing layer and/or isolating layer to obtain the gastroretentive drug carrier according to the present invention; and (2) coating a drug-containing layer on the surface of the hollow vesicle or the gastroretentive drug carrier.

In another aspect, the present invention provides a gastroretentive pharmaceutical formulation comprising the gas-vesicle-type gastroretentive drug delivery system according to the present invention and one or more pharmaceutically acceptable excipients and/or carriers.

In a further aspect, the present invention relates to the use of the gas-vesicle-type gastroretentive drug delivery system in the preparation of a medicament.

The present invention also relates to a method for preventing or treating the disease in a subject, the method comprising administering the gastroretentive pharmaceutical preparation according to the present invention to the subject. The gastroretentive pharmaceutical preparation can be administered by a variety of routes, including oral adminitration, gastric perfusion and so on.

### BRIEF DESCRIPTION OF THE DRAWINGS

Figure 1 is a schematic drawing of a gas-vesicle-type microporous osmotic pump gastric floating controlled release formulation.

Figure 2 is a schematic drawing of a gas-vesicle-type gastric floating sustained release matrix formulation.

Figure 3 is a schematic drawing of a gas-vesicle-type gastric floating membrane-controlled sustained release formulation.

Figure 4 shows a schematic drawing of a device used to measure floating-holding ability.

Figure 5 shows the release curve of rosiglitazone maleate gastroretentive controlled release formulation according to example 1.

Figure 6 shows the release curve of rosiglitazone maleate gastroretentive controlled release formulation according to example 2.

Figure 7 shows the release curve of gentamicin sulfate gastroretentive controlled release formulation according to example 3.

Figure 8 shows the release curve of gentamicin sulfate gastroretentive controlled release formulation according to example 4.

Figure 9 shows the release curve of gentamicin sulfate gastroretentive controlled release formulation according to example 5.

Figure 10 shows the release curve of gentamicin sulfate gastroretentive immediate release/sustained release formulation according to example 6.

Figure 11 shows the release curve of gentamicin sulfate gastroretentive immediate release/controlled release formulation according to example 7.

Figure 12 shows the release curve of gentamicin sulfate gastroretentive sustained release formulation according to example 8.

Figure 13 shows the release curve of ondansetron hydrochloride gastroretentive controlled release formulation according to example 9.

Figure 14 shows the release curve of ondansetron hydrochloride gastroretentive controlled release formulation according to example 10.

Figure 15 shows the release curve of ondansetron hydrochloride gastroretentive controlled release formulation according to example 11.

Figure 16 shows the release curve of ondansetron hydrochloride gastroretentive controlled release formulation according to example 12.

Figure 17 shows the release curve of ranitidine hydrochloride gastroretentive immediate release/controlled release formulation according to example 13.

Figure 18 shows the release curve of ranitidine hydrochloride gastroretentive sustained release formulation according to example 14.

Figure 19 shows the release curve of ranitidine hydrochloride gastroretentive controlled release formulation according to example 15.

Figure 20 shows the release curve of ranitidine hydrochloride gastroretentive immediate release/ sustained release formulation according to example 16.

Figure 21 shows the release curve of ranitidine hydrochloride gastroretentive controlled release formulation according to example 17.

Figure 22 shows the release curve of ranitidine hydrochloride gastroretentive immediate release/controlled release formulation according to example 18.

Figure 23 shows the release curve of ranitidine hydrochloride gastroretentive controlled release formulation according to example 19.

Figure 24 shows the plasma concentration curve of ranitidine hydrochloride gastric floating sustained release formulation according to example 14 after being administered to Beagle dogs (n=6).

Figure 25 shows the x-ray contrast of stomach of gastric floating dynamics according to example 20.

### DETAILED DESCRIPTION OF THE INVENTION

Unless otherwise indicated, the percentage used in the present invention refers to the weight percentage.

In the context of this application, the term "hollow vesicle" is used interchangeably with "gas vecisle" and refers to a gas vesicle that is hollow and has a cavity inside. However, it is necessary to point out that the "hollow vesicle" of the present invention may be completely hollow without any content, but also may contain certain pharmaceutically acceptable ingredients, such as a gas-generating agent, provided that the presence of the content will not significantly affect the density of the gastroretentive drug delivery system and thereby affect its suspension in the stomach.

In the present invention, the hollow vesicle may be made of any appropriate pharmaceutically acceptable material, provided that it can float in the stomach when a drug is loaded. The hollow vesicle can be a capsule, such as common machine-made hard capsules, including common stomach soluble capsule and intestine soluble capsule and so on. According to the difference between materials, the machine-made hard capsules include capsules made of gelatin as main raw material and capsules made of other materials, such as capsules made of plant-derived cellulose and naturally occurring water-soluble polysaccharides as raw material.

In one embodiment, the gastroretentive drug carrier or release system according to the present invention may be presented as single-chamber type, that is to say, one unit of the carrier or formulation only contains one hollow vesicle. In another embodiment, the gastroretentive drug carrier or release system according to the present invention may also be presented as multi-chamber type, that is to say, one unit of the carrier or formulation can contain two or more hollow vesicles. Preferably, the gastroretentive drug carrier or release system according to the present invention is presented as single-chamber type. There is no limitation on the shape of the hollow vesicle of the gastroretentive drug carrier or release system of the present invention. The shape can be spherical, ellipsoid, oblong or other shapes with a round and smooth surface or irregular shapes, preferably spherical or ellipsoidal or oblong. The gastroretentive drug carrier or release system according to the present invention may have any suitable size and volume. Preferably, the maximum radial size of the hollow vesicle of the gastroretentive drug delivery system is 0.5cm to 3.5cm, more preferably 0.7cm to 3.0cm, especially preferably 1.0cm to 2.5cm. For irregular shapes, the above maximum radial size refers to the maximum projection length in a certain direction. The volume of the hollow vesicle is preferably 1.1ml-1.5ml, more preferably 0.2ml-1.2ml, particularly preferably 0.3ml-0.9ml.

In the present invention, any suitable hard gelatin capsule may be used, including for example common stomach soluble capsule, intestine soluble capsule and insoluble capsule. The size of the capsule ranges from No.00# prolonged to No.5#, and other special specifications. The specific size of the capsule can be selected according to the dosage of the drug, the amount of excipients and the specific need. In this field, the sizes of empty capsules are generally divided into 00# prolonged, 00#, 0# prolonged, 0#, 1#, 2#, 3#, 4# and 5# from larger to little. The corresponding sizes of the empty capsules are usually as follows: for 00# prolonged capsule, the full closure length is 25.3±0.3mm and the outside diameter is 8.53mm (based on the capsule cap); for 00# capsule, the full closure length is 23.3±0.3mm and the outside diameter is 8.53mm (based on the capsule cap); for 0# prolonged capsule, the full closure length is 23.1±0.3mm and the outside diameter is 7.65mm (based on the capsule cap); for 0# capsule, the full closure length is 21.7±0.3mm and the outside diameter is 7.64mm (based on the capsule cap); for 1# capsule, the full closure length is 19.4±0.3mm and the outside diameter is 6.91mm (based on the capsule cap); for 2# capsule, the full closure length is 18.0±0.3mm and the outside diameter is 6.35mm (based on the capsule cap); for 3# capsule, the full closure length is 15.9±0.3mm and the outside diameter is 5.82mm (based on the capsule cap); for 4# capsule, the full closure length is 14.3±0.3mm and the outside diameter is 5.32mm (based on the capsule cap); for 5# capsule, the full closure length is 11.0±0.3mm and the outside diameter is 4.91mm (based on the capsule cap). The shape of the capsule or the common machine-made capsule is, for example, spherical, oblong or elliptic, and can also be other round and smooth shapes or irregular shapes.

In another embodiment, the gas vesicle may be a closed hollow vesicle. The gas vesicle can be made of more hydrophobic pharmaceutically acceptable polymer materials as the main raw material, such as polyethylene, polyoxyethylene, polypropylene, poloxamer and so on. Since the hydrophobicity of polymer materials is high, it is possible that the shape and the density of the gas vesicle made of said materials keep unchanged during the whole release process. Said gas vesicles can be prepared according to known techniques in the field of polymer processing, for example, injection molding, blow molding, compression molding, extrusion molding, calendering molding, plastic sucking molding and rotational molding techniques. Take injection molding for example, in general, the process is as follows: 1) placing grainy material into an injection molding machine; 2) melting the grainy material at a high temperature; 3) allowing the fluidy melt material to flow into a prepared mold; 4) cooling the material stably to mold. For polyethylene, its molding condition mainly includes cartridge temperature, mold temperature and injection pressure, for example, the cartridge temperature is generally controlled at 140 to 250°C, the mold temperature is generally controlled at 10 to 50°C, and the injection pressure is generally controlled at 50 to 100 MPa. The relevant processes and methods can be found in, for example, "Principles of Polymer Materials Processing"(Wang Guiheng, Chemical Industry Press, Beijing, 1982), "Plastics Extrusion"(Liu Ruixia, Chemical Industry Press, Beijing, 2005) and other prior art.

For the gastroretentive drug carrier or release system of the present invention, the hollow vesicle can also optionally be coated with a waterproofing layer made of polymer materials and/or hydrophobic materials, so that the film-forming polymer material and/or the hydrophobic material (for example, waxy material, such as medicinal paraffin) outside of the vesicle can effectively isolate the vesicle from gastric acid during the entire release period, to avoid the rupture of the vesicle or the change of the shape, especially for common stomach soluble capsule, since it rapidly melt and disintegrate in the stomach. Therefore, coating with a waterproofing layer on the outer surface of the capsule can achieve the purpose of effectively protecting the capsule. The hollow capsule coated with a waterproofing layer has better waterproofing performance and is more suitable for the hollow vesicle using gelatin and other hydrophilic materials as the main components. Although the shape of the intestine soluble capsules can keep intact in the stomach without coated with a waterproofing layer on the surface, however, it is apparently also appropriate for coating on its surface with a waterproof layer.

In the present invention, any pharmaceutically acceptable polymer material can be used to prepare the waterproofing layer of the gas vesicle, for example one or more polymer materials selected from ethyl cellulose, cellulose acetate, Eudragit S, Eudragit L, cellulose acetate phthalate (CAP), hydroxypropyl methylcellulose acetate succinate (HPMCAS), hydroxypropyl methyl cellulose phthalate (HPMCP), Opadry and so on. Preferably, the weight gain of the waterproofing layer is 5% to 50% by weight of the gas vesicle, more preferably 10% to 30%. Any pharmaceutically acceptable hydrophobic material may also be used to form the waterproofing layer coating the hollow capsule of the present invention, including one or more materials selected from for example cetyl alcohol, stearic acid, carnauba wax, white wax, hydrogenated castor oil and so on. Preferably, the amount of the hydrophobic material is 5% to 50% by weight of the gas vesicle, more preferably 10% to 30%.

In the case of using common machine-made capsules as the hollow vesicles of the present invention, when the required weight gain of the drug and excipients in the drug-containing layer is relatively small, the gap between the body and the cap of the capsule used may not be completely covered by the coating to form a continuous and uniform drug-containing layer, at this time, it may be advantageous to coat the capsule firstly using a pharmaceutically acceptable excipient before coating the drug-containing layer in ordet to form a continuous and smooth isolating layer, and then the drug-containing layer is coated to form a continuous coating layer in the case the weight gain is relatively small, which is advantageous to control the drug release. The presence of the isolating layer is particularly beneficial to obtain a continuous and uniform drug-containing layer, especially in the case that the amount of the drug or the excipient used is not enough to coat the whole outer surface of the capsule. It is worthy to note that the waterproofing layer and the isolating layer may exist alone or together in the gastroretentive drug carrier or release system of the present invention, of course, it is possible that neither of them exist. When the waterproofing layer and the isolating layer both exist, the isolating layer can be coated on the outer surface of the waterproofing layer, or the isolating layer can be the extension part of the waterproofing layer, if necessary, it can be a part of a capsule together with the waterproofing layer.

The isolating layer of the present invention can contain a variety of excipients, including one or more pharmaceutically acceptable excipients, such as calcium hydrogen phosphate, starch, dextrin, sucrose, lactose, mannitol, hydroxypropyl cellulose, methyl cellulose, sodium chloride, glucose, talc powder, titanium dioxide, polyethylene glycol, microcrystalline cellulose, pregelatinized starch and the like. Preferably, the weight of the isolating layer is 10% to 50% by weight of the gas vesicle, more preferably 20% to 40%.

In one embodiment of the invention, a waterproofing capsule may be prepared directly using a pharmaceutically acceptable polymer material (such as intestine soluble material) in advance. In this case, a separate waterproofing layer and/or isolating layer need not be presented on the surface of the gas vesicle. Useful polymer materials can be those materials described above for the waterproofing layer of the gas vesicle, for example, selected from ethyl cellulose, cellulose acetate, Eudragit S, Eudragit L, cellulose acetate phthalate (CAP), hydroxypropyl methylcellulose acetate succinate (HPMCAS), hydroxypropyl methyl cellulose phthalate (HPMCP), Opadry or any combination thereof.

As mentioned above, the gas vesicle used in the gastroretentive drug carrier or delivery system of the invention need not be completely hollow. Optionally, the gas vesicle used in the gastroretentive drug carrier or delivery system of the invention may contain one or more pharmaceutically acceptable substances. In one embodiment, the gas vesicle contains a gas-generating agent, for example carbonates and pharmaceutically acceptable organic acids. The carbonates include carbonates and bicarbonates, for example, sodium carbonate, sodium bicarbonate, magnesium carbonate or any combination thereof and so on, preferably sodium bicarbonate. Preferably, the gas-generating agent is a combination of sodium bicarbonate and citric acid. The amount of carbonates can be 1% to 20% by weight of the gas vesicle. In the present invention, suitable organic acids include one or more pharmaceutically acceptable organic acids, for example citric acid, malic acid, succinic acid, tartaric acid, fumaric acid, maleic acid, ascorbic acid, glutamic acid and fumaric acid. The amount of the organic acid used may be 1% to 20% by weight of the gas vesicle, more preferably 5% to 10%. After a small amount of water entering into the gas vesicle, the gas-generating agent produces carbon dioxide gas by effervescent reaction between the carbonates and the organic acids. The gas is encapsuled in the gas vesicle so that the gas does not overflow because of gastric motility to cause the change of the floatin performance, thereby playing a good supporting role on the deformed gas vesicle, and thus avoiding the impact of the floating performance caused by the shape change of the gas vesicle. In addition, the outward thrust produced by expansion of gas in the gas vesicle is constant and can effectively promote the release of insoluble drug and avoid that the drug residue in the preparation to result in a low bioavailability.

In one embodiment of the invention, any suitable drug may be coated on the hollow gas vesicle or the gastroretentive drug carrier of the present invention to be used as the drug-containing layer. The drug-containing layer may be any suitable form including conventional release layer. It can also be a sustained-release layer or a controlled-release layer. A sustained-release formulation means a formulation that releases the drug slowly and non-constantly in a prescribed medium according to the requirement. Compared with a common formulation, the sustained-release formulation can reduce the dosing frequency by a half or in some degree and can significantly increase the compliance of patients. A controlled-release formulation means a formulation that releases the drug slowly and constantly or nearly constantly in a prescribed medium according to the requirement. Compared with a common formulation, the controlled-release formulation can reduce the dosing frequency by a half or in some degree and can provide a more steady plasma drug concentration and thus can significantly increase the compliance of patients (Chinese Pharmacopoeia 2005 edition, Appendix XIX D, the guidlines for sustained-release formulations, controlled-release formulations and delayed-release formulations). "Sustained release" used herein means that the drug is constantly released for a long period after administration in order to achieve long-lasting effect, wherein the drug release process is mainly first-order release {In (1-Mₜ/M_{∞}) = -kt} or pseudo-first-order release (Mₜ/M_{∞}=kt^{1/2}) process. "Controlled release" used herein means that the drug is released at zero-order or near zero-order speed (Mₜ/M_{∞}= kt) within a predetermined time. The drug-containing layer of the present invention can optionally contain a pharmaceutically acceptable excipient or carrier. It should be appreciated by those skilled in the art to select these excipients or carriers according to the prior art. In another embodiment of the present invention, the gas vesicle may also contain a certain amount of a gas-generating agent, thus, even if water has entered into the gas vesicle, the gas produced by the gas-generating agent can still maintain the shape of the gas vesicle to ensure the buoyancy unchanged or substantively unchanged. Preferably, the gas-generating agent includes carbonate or bicarbonate salt, and pharmaceutically acceptable organic acids.

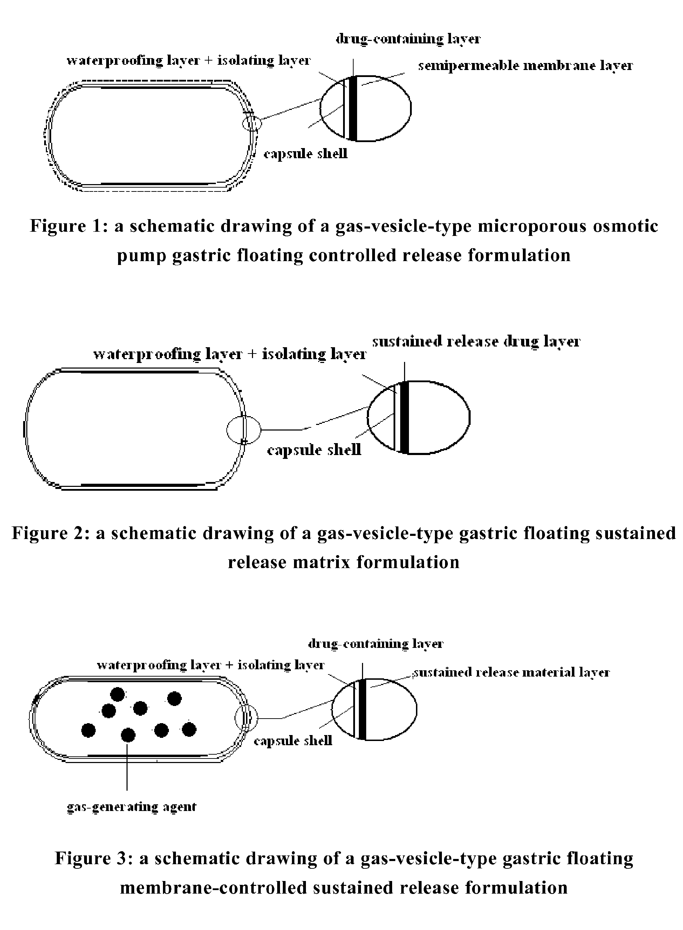

Certain illustrative structures of the gas-vesicle-type gastroretentive drug delivery system of the present invention are shown in figures 1, 2 and 3. It should be understood that the present invention is not limited to these structures.

In one embodiment of the invention, the drug may be mixed with the sustained release material and coated on the hollow vesicle or the gastroretentive drug carrier to form the drug sustained-release layer, thus, the desired sustained-release effect can be achieved. In another embodiment of the invention, after the drug-containing layer is coated on the carrier or the hollow vesicle, the sustained-release layer is coated on the drug-containing layer, and the desired sustained-release effect can also be achieved.

The sustained-release layer can be obtained by the following two ways: 1) coating the hollow vesicle or the gastroretentive drug carrier of the present invention with the drug and optional excipients, and then coating with the sustaineded-release materials to form the sustaineded-release layer, thereby a gas-vesicle-type gastric floating preparation is formed; 2) mixing the drug with the sustained release materials and optional excipients, and coating on the gas capsule or the gastroretentive drug carrier of the present invention to form directly the drug-containing sustained release layer, thereby formng a gas-vesicle-type gastric floating sustained-release matrix formulation. The above two structures can be found in figures 2 and 3. The sustained-release layer can contain a variety of excipients, such as sustained-release materials, adhesives, lubricants, penetration enhancers, solvents or any combination thereof. In one embodiment of the invention, suitable sustained-release material may be selected from one or more drug sustained-release materials, such as hydroxypropyl methyl cellulose, polyethylene oxide, ethyl cellulose, hydroxypropyl cellulose, acrylic resins, stearic acid, carnauba wax, hydrogenated castor oil. Preferably, the amount of the sustained-release material used is 10 to 70 wt/wt% of the sustained-release layer, more preferably 20 to 50 wt/wt%. Suitable adhesive can be one or more pharmaceutical adhesives selected from the group consisting of povidone, methyl cellulose, ethyl cellulose, hydroxypropyl cellulose, carboxymethyl cellulose, hydroxypropyl methyl cellulose, and the amount thereof is 5 to 30 wt/wt% of the sustained-release layer. Suitable lubricant may be one or more pharmaceutical lubricants selected from the group consisting of magnesium stearate, talc powder, polyethylene glycol, stearic acid, sodium stearyl fumarate, zinc stearate, aluminum stearate and hydrogenated castor oil, and the amount thereof is 1% to 20% by weight of the sustained-release layer, more preferably 2 to 15%. Useful penetration enhancers can be selected from the group consisting of low molecular weight sugars such as sucrose, sorbitol, mannitol, glucose, lactose, fructose; inorganic salts such as sodium chloride, potassium chloride, magnesium sulfate, potassium sulfate, sodium sulfate, or any combination thereof, and the amount thereof is 5% to 50% by weight of the sustained-release layer, preferably 10 to 30%. Any suitable cosolvent can be used in the present invention, including one or more pharmaceutical cosolvents such as polyethylene glycol, Tween-80, polyoxyethylene hydrogenated castor oil, sodium lauryl sulfate, β-cyclodextrin, hydroxypropyl β-cyclodextrin and so on. Preferably, the amount of the cosolvent is 10% to 500% by weight of the drug, preferably 100% to 200%. Additionly and optionally, a gas-generating agent may also be added in the sustained-release layer. The gas-generating agent may be selected from, for example carbonate or bicarbonate, preferably sodium carbonate, sodium bicarbonate, magnesium carbonate or any combination thereof. Preferably, the amount of the gas-generating agent used is 1% to 20% by weight of the sustained-release layer. After oral administration, the gas-generating agent produces gas in the sustained-release matrix after contacting with gastric acid and this can further reduce the density of the entire drug delivery system.

In the present invention, the drug can also be designed to release in a controlled form. In one embodiment, the drug and optional excipient will be coated in a hollow vesicle or the gastroretentive drug carrier of the invention, and then coated with the controlled-release coating to form the controlled-release layer, which together form the drug-containing layer, thus, a ideal zero-order release can be achieved, and a new type controlled-release gastric floating preparation is formed. In another embodiment, the controlled-release coating is made by film-coating with the pharmaceutically acceptable polymer. The coating contains an appropriate amount of a pore-forming agent. Micro-pores are formed when the coating contacts with water to control the drug release rate, thus a gastroretentive controlled release drug delivery system is obtained.

In the present invention, any suitable excipient can be used to prepare the controlled release layer, which consists of the drug-containing layer and the controlled-release coating film. In one embodiment, the drug-containing layer of the controlled release layer may be prepared by one or more excipients selected from adhesives, lubricants, penetration enhancers and cosolvents. The adhesive may be one or more pharmaceutical adhesives selected from the group consisting of povidone, methyl cellulose, ethyl cellulose, hydroxypropyl cellulose, carboxymethyl cellulose, hydroxypropyl methyl cellulose, and the amount thereof is 1% to 50% by weight of the drug-containing layer, preferably 5% to 30%. The lubricant used may be one or more lubricants selected from for example magnesium stearate, talc powder, polyethylene glycol, stearic acid, sodium stearyl fumarate, zinc stearate, aluminum stearate, hydrogenated castor oil. The amount thereof is 1% to 30% by weight of the drug-containing layer, preferably 3 to 20%. The penetration enhancer can be selected from one or more sugars with low molecular weight such as sucrose, sorbitol, mannitol, glucose, lactose and fructose; one or more inorganic salts selected from sodium chloride, potassium chloride, magnesium sulfate, potassium sulfate and sodium sulfate. The amount thereof is 10% to 80% by weight of the drug-containing layer, preferably 20 to 60%. The solutizer can be one or more pharmaceutical solutizers selected from for example polyethylene glycol, Tween-80, polyoxyethylene hydrogenated castor oil, sodium dodecyl sulfate, β-cyclodextrin, hydroxypropyl β-cyclodextrin. The amount thereof is 10% to 500% by weight of the drug, preferably to 100% to 200%. The controlled-release coating can be selected from such as polymer materials, plasticizers, pore-forming agents or any combination thereof, the weight gain of the controlled-release coating is 1% to 40% by weight of the whole preparation, wherein polymer materials used can be one or more polymer materials selected from cellulose acetate, ethyl cellulose, acrylic resins, hydroxypropyl methyl cellulose acetate, polyvinyl alcohol and so on, and the amount thereof is 20% to 90% by weight of the controlled-release coating, preferably 50% to 80%. The plasticizer used can be one or more plasticizers selected from glycerin, propylene glycol, polyethylene glycol, triethyl citrate and phthalate esters, and the amount thereof is 5% to 40% by weight of the controlled-release coating, preferably 10% to 30%. The pore-forming agent can be one or more soluble substances selected from glycerin, propylene glycol, polyethylene glycol, sucrose, mannitol, lactose, sodium chloride, hydroxypropyl cellulose, methyl cellulose, hydroxypropyl methyl cellulose, povidone and so on, and the amount thereof is 5% to 40% by weight of the controlled-release coating, preferably 10% to 30%. An example of the gas-vesicle-type gastroretentive controlled-release formulations is microporous gas-vesicle-type gastric floating osmotic pump controlled-release preparation, whose structure is shown in Figure 1.

By adopting a sustained-release or controlled release coating, the release rate is effectively controlled, meanwhile the weight and volume of the formulation do not significantly increase, thus, it is more advantagous for packaging and transport and more easy for patients to swallow. In addition, the formulation itself is a sustained/controlled release formulation and can maintain a constant release rate after being discharged from the stomach into the intestine. For drugs absorbed poorly in colon, the residence time in the absorption site can be effectively prolonged, thereby enabling administration once every 24h, 36h or longer even 48 hours. Therefore, the compliance of patients is greatly increased.

If necessary, the gastroretentive drug delivery system of the present invention may also contain an immediate release layer. Dual-rate release can be achieved by coating the sustained/controlled release layer with an immediate release layer, that is to say, an appropriate initial drug concentration is provided at the initial stage of administration, and then a constant release rate is maintained, thereby maintaining a stable plasma concentration. The immediate release layer consists of drug and an optional excipients and is coated on the sustained release layer or the controlled release layer to form an immediate release layer, and thus a double-rate gastroretentive sustained/controlled release drug delivery system is prepareed. The excipient may optionally include adhesives, lubricants or combination thereof. The adhesive can be one or more pharmaceutical adhesives such as povidone, methyl cellulose, ethyl cellulose, hydroxypropyl cellulose, carboxymethyl cellulose and hydroxypropyl methyl cellulose. The amount thereof is 5% to 30% by weight of the immediate-release coating layer. The lubricant may be one or more pharmaceutical lubricants selected from magnesium stearate, talc powder, polyethylene glycol, stearic acid, sodium stearate fumarate, zinc stearate, aluminum stearate and hydrogenated castor oil. The amount thereof is 1% to 30% by weight of the immediate-release coating layer.

The drugs suitable for the gastroretentive drug delivery systems of the present invention include all drugs appropriate for being prepared into gastroretentive formulations, for example: 1) anti-acid drugs that play a role in the stomach; 2) drugs that are mainly absorbed in the stomach, such as weak organic acid drugs; 3) drugs whose solubility in the stomach is greater than that in the intestine; 4) drugs with particular requirement in clinic, such as the gastric floating preparations coated with anhydrous citric acid and sodium bicarbonate as an effervescent agent used for patients with gastric cancer in which less gastric acid is secreted; and 5) the drugs those are absorbed poorly or not absorbed in the lower gastrointestinal tract and have a short half-life, which can be prepared into the gastric floating formulations so as to administer once a day.

For example, the drugs that are useful in the gastroretentive drug delivery system of the present invention can be selected from for example the following drugs or any combination thereof: hangover medicine, drugs used to treat dementia, anesthetics, drugs used to treat acromegaly, analgesics, drugs used to treat asthma, anticancer drugs, anticoagulants, antithrombotic drugs, antiepileptic drugs, diabetes drugs, antiemetic drugs, drugs used to treat glaucoma, anti-histamine drugs, anti-infective drugs, drugs used to treat Parkinson's disease, antiplatelet drugs, anti-rheumatic drugs, anti-spasm and cholinergic drugs, antitussives, carbonic anhydrase inhibitors, cardiovascular drugs, cholinesterase inhibitors, drugs used to treat central nervous system disorders, central nervous system stimulants, contraceptives, drugs used to treat cystic fibrosis, dopamine receptor agonists, drugs used to treat endometritis, drugs used to treat erectile dysfunction, drugs used to treat infertility, gastrointestinal drugs, immuno-modulatory agents, immuno-suppressive agents, drugs used to enhance memory, migraine preparations, muscle relaxation drugs, nucleoside analogues, drugs used to treat osteoporosis, drugs used to treat parasympathomimetics, prostaglandins, psychotropic drugs, sedatives, hypnotics and tranquillizers, drugs used to treat skin diseases, steroids and hormones.

In some embodiments of the present invention, the drugs are selected from rosiglitazone, gentamicin, ondansetron, ranitidine or any combination thereof. The ordinary skilled in the art should understand, besides their parent drug forms, these drugs also include pharmaceutically acceptable salts or esters or prodrugs thereof. Other selectable drugs include for example baclofen, atorvastatin calcium, faropenem sodium or any combination thereof, and pharmaceutically acceptable salts or esters or prodrugs thereof.

It should be noted that one or more drugs that are useful to treat one or more diseases can be used together in the gastroretentive drug delivery system of the invention. For example, in the treatment of gastric ulcer, clarithromycin can be used in combination with amoxicillin and omeprazole if necessary. In the treatment of cancer, chemotherapy drugs can be used in combination with antiemetics such as ondansetron. Other possible forms of drug combination used in the actual clinical practice are also included.

The gastroretentive drug delivery system of the present invention can be prepared by any suitable method. In one embodiment, the method comprises: (1) preparing or providing a hollow gas vesicle, and optionally coating the hollow gas vesicle with a waterproofing layer and/or isolating layer to obtain the gastroretentive drug carrier; and (2) coating a drug-containing layer on the surface of the above mentioned hollow gas vesicle or gastroretentive drug carrier.

In the present invention, the hollow gas vesicle can be prepared by a variety of methods, including some methods known in the prior art, such as conventional film-coating method [(Cole, G., Hogan, H., Aulton, M., Pharmaceutical Coating Technology. Chinese Medicine Science and Technology Press, 2003, Beijing, translated by Junmin Zheng). In one embodiment, the gas capsule is prepared by two-stage coating in combination with hot-melt sealing, allowing the tightness of the gas capsule to meet the design requirements and to effectively prevent the entry of water, so that the shape of the gas capsule remains unchanged. For example, the preparation steps may include: the shell of the unclosed capsule is coated with a mixed coating solution of a polymer material and a hydrophobic material. The coating process is stopped when the weight is gained to about 20% to 70% (preferably 30%) of the expected total weight gain of coating. The capsule is closed after being fully dried, then the remainder of 30% to 80 % of the coating is coated. After being coated completely, the capsule is placed under 45°C for 12 to 24 hours. After hot-melt sealing, a complete gas capsule is obtained. It showed after test that the gas capsule kept intact and unsoftened after rotation in 0.1N HCl solution at 37 °C for 6h. The total weight gain of such a waterproofing layer was 10% to 80% by weight of the capsule, preferably 20% to 60%. In another embodiment of the invention, the waterproofing gas capsule prepared in advance from a polymer material was directly used.

The drug-containing layer may be coated on the gas capsules or the drug carriers by any suitable method. Such methods are known in the prior art. For example, the drug-containing layer can be prepared using ordinary coating technology or centrifugal granulation technology, that is to say, the gas capsules are coated with the drug and optional excipients and then coated with a sustained release material to form the sustained release layer, or the gas capsules are coated with the mixture of the drug and the excipients containing a sustained release material to form the sustained release layer, thereby obtaining the gastroretentive sustained release drug delivey system; or the gas capsules are coated with the drug and optional excipients, and then coated with controlled-release coating film to form the controlled-release layer. The controlled-release coating film is prepared by film coating using a pharmaceutically acceptable polymer, and a pore-forming agent that forms micropores for the release of the drug upon contacting with water is contained in the film to control the drug release rate, thereby obtaining the gastroretentive sustained release drug delivey system.

If a double-release gastroretentive drug release system is needed to prepare, the immediate release layer can be prepared using ordinary coating technology or centrifugal granulation technology, then the drug and optional excipient such as adhesive, lubricant, are coated on the surface of the sustained release layer or the controlled release layer to form the immediate release drug-containing layer. Specific operations can be carried out by the skilled in the art according to the requirements of existing coating technology of publicly known technology. The type and amount of excipients used can be choosed in accordance with the above instructions.

In the above step (1), for the drug of smaller dosage specification, the amount of drug contained in the drug-containing layer and the weight gain required for the excipients are usually retatively small. The gap between the capsule body and capsule cap can not be completely covered by the coating to form a continuous and uniform drug-containing layer, at this time, the gas capsules should be firstly coated with a pharmaceutically acceptable excipient before coated with the drug-containing layer, in order to form a continuous and smooth isolating layer, then coated with the drug-containing layer. By this means, in the case that the weight gain is small, a continuous drug-containing layer can be formed and it is highly advantageous to control the release of the drug. The isolating layer can be the extension of the waterproofing layer and can form a part of the gas capsule together with the waterproofing layer if needed. The weight of the isolating layer is gained by 10% to 40% of the gas capsule, preferably 15% to 35%.

When the amount of drug contained in the drug-containing layer and the weight gain required for the excipients are usually retatively large, the intestine soluble capsule shells can be directly used as hollow capsules without coated with a waterproofing layer, but coated directly with the drug-containing layer. When the weight of the drug-containing layer increases to a certain value, a continuous and smooth drug-containing layer can be obtained. Then coating with the sustained release layer or controlled release coating film may be performed to get the gastroretentive sustained release/controlled release drug delivery system.

The present invention also relates to a gastroretentive pharmaceutical preparation, comprising the gastroretentive drug delivery system of the present invention and a pharmaceutically acceptable carrier and/or excipient. The carriers or excipients include for example adhesives, fillers, penetration enhancers, lubricants, plasticizers, pore-forming agents, film-forming agents (polymer materials and hydrophobic materials), gas-generating agents (carbonates and organic acids) and so on.

The present invention also provides the use of the gastroretentive drug delivery system in the preparation of a medicament for the treatment of the disease in a subject. The subject may be any vertebrate, particularly mammals, such as cattle, sheep, dog, cat, etc., especially human.

The present invention also relates to a method for treating the disease in a subject, comprising administering the gastroretentive pharmaceutical preparation of the present invention to the subject in need thereof. The gastroretentive pharmaceutical preparation of the present invention can be administered by any suitable routs, including for example oral administration, stomach feeding tube, perfusion, etc. The effective amount can be easily determined by physicians based on the general knowledge.

Compared with gastric floating formulations in the prior art, the present invention has one or more of the following advantages: (1) effectively reducing the density of the whole formulation so as to reduce the possibility of gastric discharge; (2) rapid floating initiation; (3) sustained and constant floating holding ability, due to relatively closed gas vesicle and no aptness of loss of gas. The results of the gastric floating kinetics test showed that the average gastric residence time of the gas-vesicle-type gastric floating forulation of the invention was 5h, which was much longer than the mean gastric residence time of common formulations reported in the literature (1.5h), thus, it fully met the requirements for gastric floating formulations; and (4) the method for preparing the gas-vesicle-type gastroretentive drug delivery system and the formulation according to the invention is simple. The gas capsules can be prepared using exsiting widely commercially available empty capsules and well established coating technology in the field, so the cost is low and it is easy to produce industrially.

Now, the present invention will be further described in detail in combination with the following examples and with reference to drawings in order to enable the ordinary skilled in the art more clearly understand and implement the present invention. However, it should be understood that the invention is not limited to specific circumstances and details described in these examples. Throughout this description, any and all of the publicly available references, including patent applications and issued patents, are incorporated herein by reference. It should also be understood by the ordinary skilled in the art that any change, modification and replacement made without departing from the spirit and scope of the present invention falls within the scope of the present invention.

The following examples are merely used to demonstrate the feasibility of the technical solutions of the present invention. In principle, according to the present invention, any drug suitable to be formulated into gastric floating formulation can be prepared into the gas-vesicle-type gastric floating formulation with reference to the methods described below. The common hard gelatin capsules and intestine soluable capsules used in the examples of the present invention were provided by Suzhou Capsugel Co., Ltd.

### Example

### Example 1: Gastroretentive formulation of rosiglitazone

Rosiglitazone is a second-generation thiazolidinedione hypoglycemic agent. Gastric floating tablets of rosiglitazone reported in the literature are prepared by conventional preparation techniques for gastric floating tablets as follows: the hydrophilic gel material with high viscosity, i.e, hydroxypropyl methyl cellulose (HPMC) is selected, to which appropriate amount of foaming agent is added, and then the resulting mixture is compressed into tablets by direct powder compression. The resulting gastric floating tablets are claimed to have the same bioavailability with common tablets administered in a multiple doses [Hao Feng, Zhimin Wang, Dawei Chen, Studies on floating sustained release tablets of rosiglitazone maleate, Journal of China Pharmaceutical University, 2002, 33 (3): 196-199]. However, the test results of the floating capacities of the gastric floating sustained release tablets verified by isotope scintigraphyshowed that the gastric floating tablets can not immediately float at the initial stage after administration, because the initial density is large, it will take some time for the tablets to expand, and their floating capacities will decrease with the reduce of the volume of tablet cores eroded, then the tablets are discharged from the stomach. Our study showed that the solubility of rosiglitazone in artificial gastric juice is about a million times more than that in artificial intestinal fluid, suggesting that once the common sustained release tablets or gastric floating tablets are discharged from stomach into intestinal neutral or alkaline environment, the dissolution and absorption of the drug will be significantly reduced, resulting in the bioavailability of the tablests can not be achieved in the same level as common formulations. The gas-vesicle-type floating formulation of the present invention can ensure that the density of the formulation is relatively small (generally<0.6g/cm³), thereby it can float at the early stage of administration, since the shape of the capsule keeps unchange in the stomach. Thus, the entire volume of the formulation remains relatively stable, so that stable and sustainable floating capacity is achieved.

### Gas vesicle: 1# common hard gelatin capsules

The composition of the coating solution for the waterproofing layer:

| | |
|---|---|
| Eudragit L100 | 9.0g |
| stearic acid | 15.0g |
| triethyl citrate | 4.5g |
| Talc powder | 3.0g |
| anhydrous ethanol | to 300ml |

The composition of the coating solution for the drug-containing layer:

| | |
|---|---|
| rosiglitazone maleate | 1.5g |
| Eudragit RL100 | 4.5g |
| ethyl cellulose | 2.0g |
| polyethylene glycol | 1.0g |
| 90% ethanol solution | to 100ml |

The composition of the coating solution for the controlled release film:

| | |
|---|---|
| Eudragit RS100 | 2.5g |
| ethyl cellulose | 2.0g |
| polyethylene glycol | 1.0g |
| triethyl citrate | 0.8g |
| 90% ethanol | to 100ml |

### Preparation method:

1# capsule shells were placed in a coating pan. The temperature of tablet bed was 45°C. The coating solution for the waterproofing layer (prepared by dissolving/dispersing stearic acid, Eudragit L100, triethyl citrate and talc powder in anhydrous ethanol) was sprayed into the coating pan. After the weight gain of the coating reached 15%, the capsule was locked. Continue with the coating till the weight gain reached 15%. The resulting capsule was placed at 45°C for 12h to give a waterproofing gas vesicle. Rosiglitazone maleate, Eudragit RL100, ethyl cellulose and polyethylene glycol were dissolved in 90% ethanol solution to prepare the coating solution for the drug-containing layer. The capsules prepared were placed in the coating pan. The drug-containing layer was coated on the outer surface of the gas vesicle using conventional film coating method. The weight gain of the coating was calculated based on the weight of rosiglitazone. Each capsule contained 8mg of rosiglitazone. Eudragit RS100, ethyl cellulose, polyethylene glycol and triethyl citrate were dissolved in 90% ethanol solution to prepare the coating solution for the controlled release film. The controlled release coating was coated on the outer surface of the gas vesicle using conventional film coating method. The weight gain of the coating was 24% to 26%.

### Determination of the floating-holding ability:

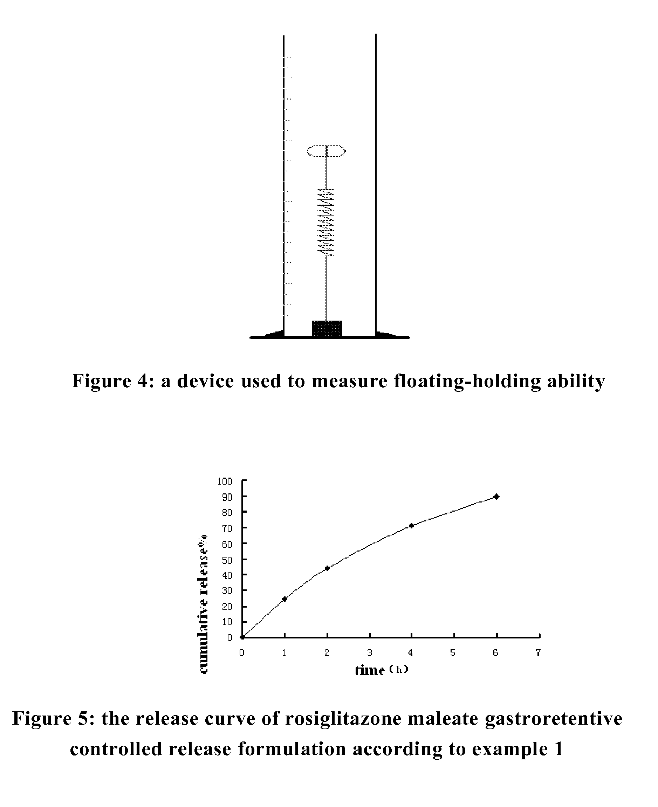

The gastroretentive formulation was fixed on the top of the spring of the floating-holding ability detector as shown in Figure 4 and placed in a measuring cup. 0.1N hydrochloric acid was added. The suspending position of the formulation in the measuring cup was recorded every hour for 12 hours. The results showed that the floating ability of the gastric floating formulation prepared in this example maintained stable and did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 1, 2, 4 and 6h respectively. The cumulative release curve was shown in Figure 5. The release curve showed that the controlled release exhibited zero-order release and the sustained release complied with Higuchi equation.

The in vitro release test also showed that the floating formulation kept floating during the entire release phase and the shape kept intact.

### Example 2

### Gas vesicles: 1# common hard gelatin capsules (1000 capsules)

The composition of the coating solution for the waterproofing layer:

| | |
|---|---|
| ethyl cellulose | 6.0g |
| stearic acid | 2.0g |
| Eudragit L | 10.0g |
| triethyl citrate | 4.5g |
| talc powder | 3.0g |
| anhydrous ethanol | to 300ml |

The composition of the coating solution for the drug-containing layer:

| | |
|---|---|
| rosiglitazone maleate | 8g |
| povidone k30 | 3g |
| mannitol | 20g |
| polyethylene glycol | 5g |
| talc powder | 3g |
| 30% ethanol | to 200ml |

The composition of the coating solution for the controlled release film:

| | |
|---|---|
| ethyl cellulose | 12g |
| polyethylene glycol 4000 | 4g |
| HPMC | 2g |
| diethyl phthalate | 1.5ml |
| 80% ethanol solution | to 400ml |

### Preparation method:

1# capsule shells were placed in a coating pan. The temperature of tablet bed was 45°C. The coating solution for the waterproofing layer (prepared by dissolving/dispersing Eudragit L100, stearic acid, ethyl cellulose, triethyl citrate and talc powder in anhydrous ethanol) was sprayed into the coating pan. After the weight gain of the coating reached 15%, the capsule was locked. The coating was performed again until the weight gain of the coating reached 15%. The resulting product was placed at 45°C for 12h to give an intestine soluble waterproofing gas vesicle. Rosiglitazone maleate, mannitol, polyethylene glycol and povidone k30 were dissolved in 30% ethanol solution to prepare the coating solution for the drug-containing layer. The gas vesicles prepared was placed in the coating pan. The drug-containing layer was coated on the outer surface of the gas vesicle using conventional film coating method. The weight gain of the coating was calculated based on the weight of rosiglitazone. Each capsule contained 8mg rosiglitazone. The coating solution for the controlled-release layer prepared was coated on the drug-containing gas vesicle using the same method to form the controlled-release film. The weight gain of the coating was 18% to 20%. The resulting product was placed at 45°C for 24h to give the gas-vesicl-type gastric floating controlled-release formulation of rosiglitazone maleate.

### Determination of the floating-holding ability

The floating-holding ability was determined according to the method of example 1. The results showed that the floating ability of the gastric floating formulation prepared in this example maintained stable and did not decrease within 12 hours.

### In vitro release test

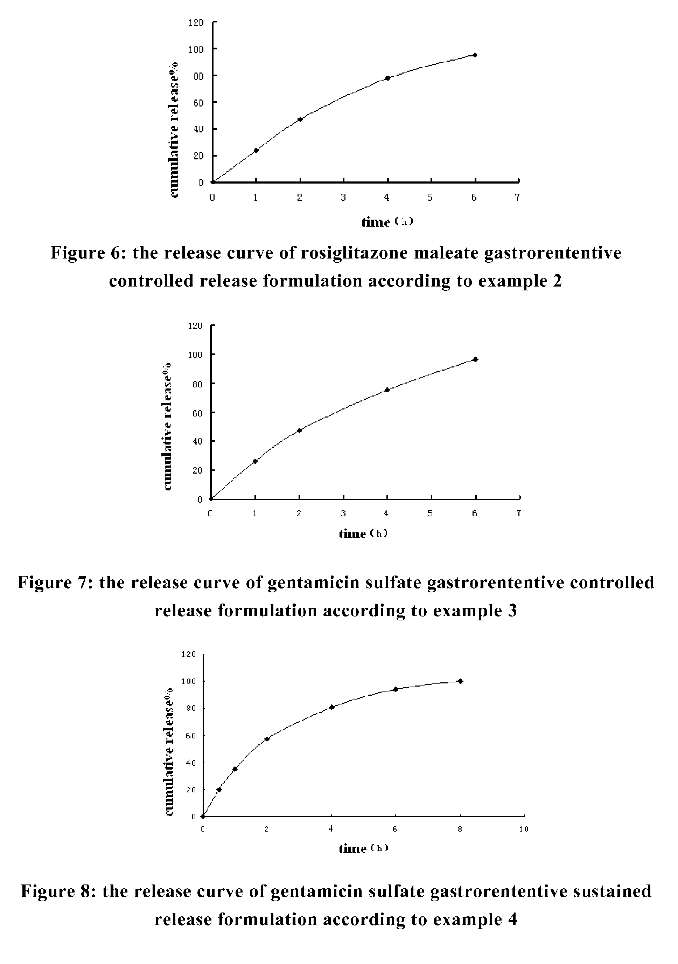

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 1, 2, 4 and 6h respectively. The cumulative release curve was shown in Figure 6. The release curve showed that the controlled release exhibited zero-order release and the sustained release complied with Higuchi equation.

The in vitro release test also showed that the floating formulation kept floating during the entire release phase and the shape kept intact.

### Example 3

### Gas vesicles: 1# intestine soluble capsules (1000 capsules)

The composition of the coating solution for the drug-containing layer:

| | |
|---|---|
| gentamicin sulfate | 40g |
| Eudragit RL100 | 45g |
| ethyl cellulose | 20g |
| polyethylene glycol | 10g |
| 90% ethanol solution | to 1000ml |

The composition ofthe coating solution for the controlled release film:

| | |
|---|---|
| Eudragit RS100 | 2.5g |
| ethyl cellulose | 2.0g |
| polyethylene glycol | 0.8g |
| triethyl citrate | 0.8g |
| 90% ethanol | to 100ml |

### Preparation method:

Gentamicin sulfate, Eudragit RL100, ethyl cellulose and polyethylene glycol were dissolved in 90% ethanol solution to prepare the coating solution for the drug-containing layer. 1# intestine soluble capsule shells were placed in a coating pan. The drug-containing layer was coated on the outer surface of the gas vesicle using conventional film coating method. The weight gain of the coating was calculated based on the weight of gentamicin. Each capsule contained 40mg gentamicin. The coating solution for the controlled-release layer prepared was coated on the drug-containing gas vesicle using the same method to form the controlled-release film. The weight gain of the coating was 15% to 18%. The resulting product was placed at 45°C for 24h to give the gas-vesicl-type gastric floating controlled-release formulation of gentamicin sulfate.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 7.

### Example 4

### Gas vesicles: 0# common hard gelatin capsules (1000 capsules)

The gas-generating agent consisted of 7.5g of citric acid and 5g of sodium bicarbonate.

The composition ofthe coating solution for the waterproofing layer:

| | |
|---|---|
| Eudragit RS | 15g |
| stearic acid | 9.0g |
| triethyl citrate | 4.5g |
| talc powder | 3.0g |
| anhydrous ethanol | to 400ml |

The composition of the coating solution for the drug-containing layer:

| | |
|---|---|
| gentamicin sulfate | 40g |
| povidone k30 | 40g |
| lactose | 30g |
| talc powder | 4g |
| anhydrous ethanol | to 600ml |

The composition of the coating solution for the sustained release film:

| | |
|---|---|
| Eudragit RS | 10g |
| Eudragit RL | 3g |
| polyethylene glycol 4000 | 1.5g |
| diethyl phthalate | 1.5ml |
| 80% ethanol solution | to 400ml |

### Preparation method:

Prescribing amount of sodium bicarbonate and citric acid were encapsuled in 1# capsule shell and placed in a coating pan. The temperature of tablet bed was 45°C. The coating solution for the waterproofing layer (prepared by dissolving/dispersing Eudragit RS, triethyl citrate, stearic acid and talc powder in anhydrous ethanol) was sprayed into the coating pan. After the weight gain of the coating reached 15%, the capsule was locked. The coating was performed again until the weight gain of the coating reached 15%. The resulting product was placed at 45°C for 12h to give a gas vesicle. Gentamicin sulfate, lactose, povidone k30 and talc powder were suspended in ethanol solution to prepare the coating solution for the drug-containing layer. The drug-containing layer was coated on the outer surface of the gas vesicle using conventional film coating method. The weight gain of the coating was calculated based on the weight of gentamicin. Each capsule contained 40mg gentamicin. The coating solution for the sustained-release layer was coated on the drug-containing gas vesicle using the same method to form the sustained-release film. The weight gain of the coating was 15% to 18%. The resulting product was placed at 45°C for 24h to give the gas-vesicl-type gastric floating controlled-release formulation of gentamicin sulfate.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4, 6 and 8h respectively. The cumulative release curve was shown in Figure 8. The results showed that the gastroretentive drug delivery system of the present invention floated immediately in the in-vitro release medium, and the gas-generating agent produced bubbles when contacting with water entered in the capsule, allowing the formulation to continue expansion, and the density further decreased. The shape of the formulation remained intact during the release process and the floating-holding ability steadily increased by using membrane-control technique.

### Example 5

### Gas vesicles: 0# common hard gelatin capsules (1000 capsules)

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |
| 80% ethanol solution | to 300ml |

The composition of the coating solution for the drug-containing layer:

| | |
|---|---|
| gentamicin sulfate | 30g |
| povidone k30 | 40g |
| lactose | 30g |
| talc powder | 8g |
| 70% ethanol | to 500ml |

The composition of the coating solution for the sustained release film:

| | |
|---|---|
| Eudragit RS | 15g |
| Eudragit RL | 10g |
| triethyl citrate | 5ml |
| talc powder | 5g |
| polyethylene glycol 4000 | 10g |
| anhydrous ethanol | to 600ml |

### Preparation method:

0# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Gentamicin sulfate, lactose, talc powder and povidone k30 were suspended in ethanol solution to prepare the coating solution for the drug-containing layer. The drug-containing layer was coated on the outer surface of the gas vesicle using conventional film coating method. The weight gain of the coating was calculated based on the weight of gentamicin. Each capsule contained 40mg gentamicin. The coating solution for the sustained-release layer was coated on the drug-containing gas vesicle using the same method to form the sustained-release film. The resulting product was placed at 45 °C for 24h to give the vesicl-type gastric floating sustained-release formulation of gentamicin sulfate.

### Determination of the floating-holding ability

The floating-holding ability was determined according to the method of example 1. The results showed that the floating ability of the gastric floating formulation prepared in this example did not decrease within 12 hours.

### In vitro release test:

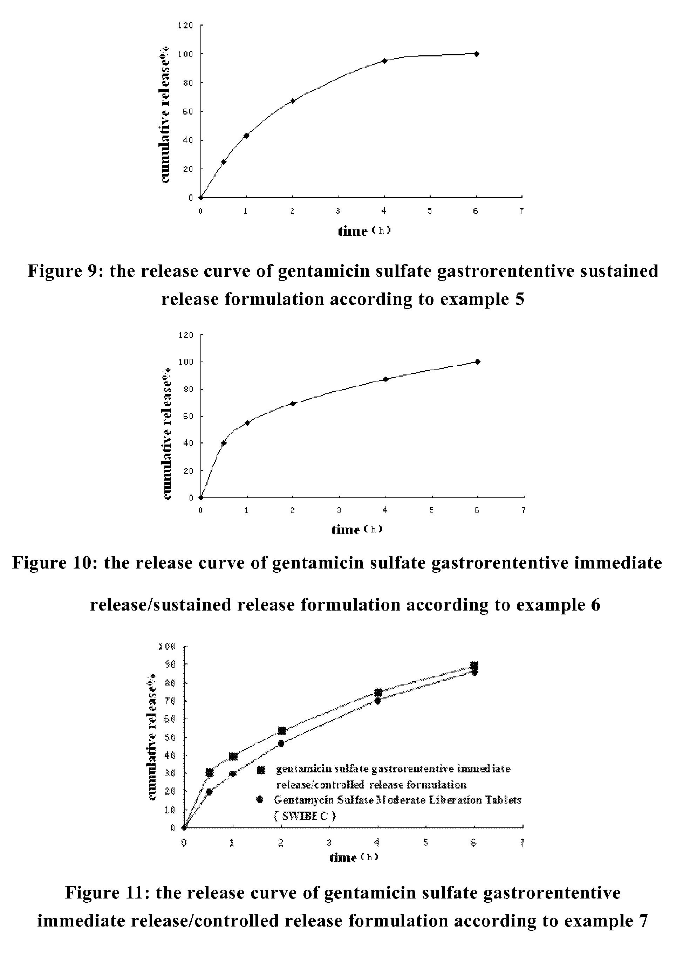

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 9.

### Example 6

### Gas vesicles: 0# common hard gelatin capsules (1000 capsules)

The composition of the coating solution for the waterproofing layer:

| | |
|---|---|
| ethyl cellulose | 18.0g |
| stearic acid | 12.0g |
| triethyl citrate | 7.5g |
| talc powder | 5.0g |
| 80% ethanol | to 500ml |

The composition of the coating solution for the drug-containing layer:

| | |
|---|---|
| gentamicin sulfate | 30g |
| povidone k30 | 30g |
| lactose | 30g |
| magnesium stearate | 4g |
| 70% ethanol solution | to 500ml |

The composition of the coating solution for the sustained release film:

| | |
|---|---|
| Eudragit RS | 18g |
| Eudragit RL | 10g |
| triethyl citrate | 5g |
| talc powder | 5g |
| 80% ethanol | to 500ml |

The composition of the coating solution for the immediate release layer:

| | |
|---|---|
| gentamicin sulfate | 10g |
| povidone k30 | 6g |
| talc powder | 1g |
| titanium dioxide | 1g |
| 70% ethanol solution | to 200ml |

### Preparation method:

0# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Gentamicin sulfate, lactose, and povidone k30 were dissolved in 70% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of gentamicin. Each capsule contained 40mg gentamicin. The coating solution for the sustained-release layer prepared was coated on the drug-containing gas vesicle using the same method to form the sustained-release film. After The resulting product was placed at 45°C for 24h, the immediate release coating solution prepared was coated on the outer surface of the sustained-release film to give the gas-vesicl-type gastric floating dual-release formulation of gentamicin sulfate.

### Determination of the floating-holding ability

The floating-holding ability was determined according to the method of example 1. The results showed that the gastric floating formulation prepared in this example did not decrease within 12 hours.

### In vitro release test

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 10.

### Example 7

### Gas vesicles: 0# common hard gelatin capsules (1000 capsules)

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |
| 80% ethanol solution | to 300ml |

The composition of the coating solution for the drug-containing layer:

| | |
|---|---|
| gentamicin sulfate | 30g |
| Eudragit RL100 | 45g |
| ethyl cellulose | 20g |
| polyethylene glycol | 10g |
| 90% ethanol solution | to 1000ml |

The composition of the coating solution for the controlled release film:

| | |
|---|---|
| Eudragit RS100 | 10g |
| ethyl cellulose | 10g |
| polyethylene glycol | 1.0g |
| triethyl citrate | 0.8g |
| 90% ethanol | to 100ml |

The composition of the coating solution for the immediate release layer:

| | |
|---|---|
| gentamicin sulfate | 10g |
| povidone k30 | 6g |
| talc powder | 2g |
| 70% ethanol solution | to 200ml |

### Preparation method:

0# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Gentamicin sulfate, Eudragit RL100, ethyl cellulose and polyethylene glycol were dissolved in 90% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of gentamicin. Each capsule contained 40mg gentamicin. The coating solution for the controlled-release film was coated on the drug-containing gas vesicle using the same method to form the controlled-release coating. The weight gain of the coating was 15% to 18%. After The resulting product was placed at 45°C for 24h, the immediate release coating solution prepared was coated on the outer surface of the controlled-release film to give the gas-vesicl-type gastric floating dual-release formulation of gentamicin sulfate.

### Determination of the floating-holding ability:

The floating-holding ability was determined according to the method of example 1. The results showed that the gastric floating formulation prepared in this example did not decrease within 12 hours. In the determination of the floating-holding ability, the gastric floating sustained release tablets of gentamicin sulfate (SWIBEC) as a control floated after 1h. SWIBEC settled again at 4h since the sustained-release materials was dissolved and the volume of the sustained release tablet decreased.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. At the same time, release test was performed for the gastric floating sustained release tablets of gentamicin sulfate (SWIBEC). The cumulative release curve was shown in Figure 11.

### Example 8

### Gas vesicles: 0# common hard gelatin capsules (1000 capsules)

The composition of the coating solution for the waterproofing layer:

| | |
|---|---|
| ethyl cellulose | 12.0g |
| stearic acid | 20.0g |
| triethyl citrate | 6.0g |
| talc powder | 4.5g |
| 80% ethanol solution | to 600ml |

The comnosition of the coating solution for the drug-containing layer:

| | |
|---|---|
| gentamicin sulfate | 40g |
| polyoxyethylene | 15 g |
| stearic acid | 15 g |
| sodium bicarbonate | 6g |

### Preparation method:

0# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Gentamicin sulfate, polyoxyethylene and stearic acid were screened with a 100-mesh sieve and mixed well to form the coating powder for the dry method. The waterproofing gas vesicles prepared above were placed in the coating pan. A sustained-release drug-containing layer was coated on the outside surface of the waterproofing gas vesicles using centrifugal granulation technology to give the vesicl-type gastric floating sustained-release formulation of gentamicin sulfate. The weight gain of the coating was calculated based on the weight of gentamicin. Each capsule contained 40mg gentamicin.

### Determination of the floating-holding ability;

The floating-holding ability was determined according to the method of example 1. The results showed that the gastric floating formulation prepared in this example did not decrease within 12 hours.

### In vitro release test:

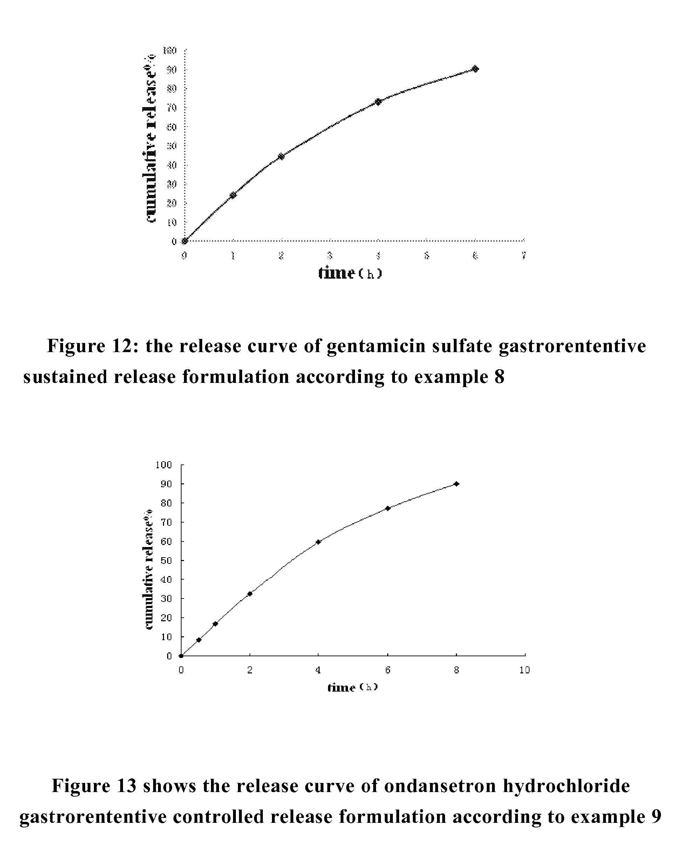

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 12.

### Example 9

### Gas vesicles: 1# common hard gelatin capsules (1000 capsules)

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |

The composition of the coating solution for the drug-containing layer:

| | |
|---|---|
| ondansetron hydrochloride | 8.0g |
| povidone k30 | 4.0g |
| lactose | 20.0g |
| talc powder | 6.0g |
| 30% ethanol solution | to 200ml |

The composition of the coating solution for the controlled release film:

| | |
|---|---|
| cellulose acetate | 12.0g |
| polyethylene glycol 4000 | 2.0g |
| diethyl phthalate | 1.5ml |
| acetone/ethanol/water (14/5/1) | to 400ml |

### Preparation method:

1# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Ondansetron hydrochloride, lactose, povidone k30 and talc powder were dissolved/dispersed in 30% ethanol solution to prepare the coating solution for the drug-containing layer. The gas vesicles prepared above were placed into the coating pan. The drug-containing layer was coated on the outer surface of the gas vesicle using conventional film coating method. The weight gain of the coating was calculated based on the weight of ondansetron. Each capsule contained 8mg ondansetron. The coating solution for the controlled-release layer was coated on the drug-containing gas vesicle using the same method to form the controlled-release film. The weight gain of the coating was 6%. After The resulting product was placed at 45°C for 24h, the gas-vesicl-type gastric floating controlled release formulation of ondansetron hydrochloride was obtained.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4, 6 and 8h respectively. The cumulative release curve was shown in Figure 13. It can be seen that ondansetron gastroretentive formulation can effectively extend the residence time of drugs in the gastrointestinal tract and improve bioavailability.

### Example 10

### Gas vesicles: 1# common hard gelatin capsules (1000 capsules)

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |
| anhydrous ethanol | to 300ml |

The composition of the coating solution for the isolating layer:

| | |
|---|---|
| lactose | 30g |
| povidone k30 | 3.0g |
| anhydrous ethanol | 200ml |

The composition of the coating solution for the drug-containing layer:

| | |
|---|---|
| ondansetron hydrochloride | 1.5g |
| Eudragit RL100 | 5.0g |
| ethyl cellulose | 2.0g |
| polyethylene glycol | 1.0g |
| 90% ethanol solution | 100ml |

The composition of the coating solution for the controlled release film:

| | |
|---|---|
| Eudragit RS100 | 2.5g |
| ethyl cellulose | 2.0g |
| polyethylene glycol | 0.8g |
| triethyl citrate | 0.8g |
| 90% ethanol solution | 100ml |

### Preparation method:

1# capsule shells were placed in a coating pan. The temperature of tablet bed was 45°C. The coating solution for the waterproofing layer (prepared by dissolving/dispersing stearic acid, ethyl cellulose, triethyl citrate and talc powder in anhydrous ethanol) was sprayed into the coating pan. After the weight gain of the coating reached 15%, the capsule was locked. The coating was performed again until the weight gain was 20%. The resulting product was placed at 45°C for 12h to give a waterproofing gas vesicle. Lactose was dispersed in an anhydrous ethanol solution containing povidone k30 to give a coating suspension of the isolating layer. The gas vesicles prepared above were placed in the coating pan. The isolating layer was coated on the outer surface of the gas vesicle using conventional film coating method. The weight gain of the coating was 25%. Ondansetron hydrochloride, Eugragit RL100, ethyl cellulose and polyethylene glycol were dissolved in 90% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of ondansetron. Each capsule contained 8mg ondansetron. Eugragit RS100, ethyl cellulose, polyethylene glycol and triethyl citrate were dissolved in 90% ethanol solution to prepare the coating solution for the controlled release film. The controlled release film was coated on the outer surface of the drug-containing gas vesicle using conventional film coating method. The weight gain of the coating was 15%-17%.

### Determination of the floating-holding ability:

The floating-holding ability was determined according to the method of example 1. The results showed that the gastric floating formulation prepared in this example did not decrease within 12 hours.

### In vitro release test:

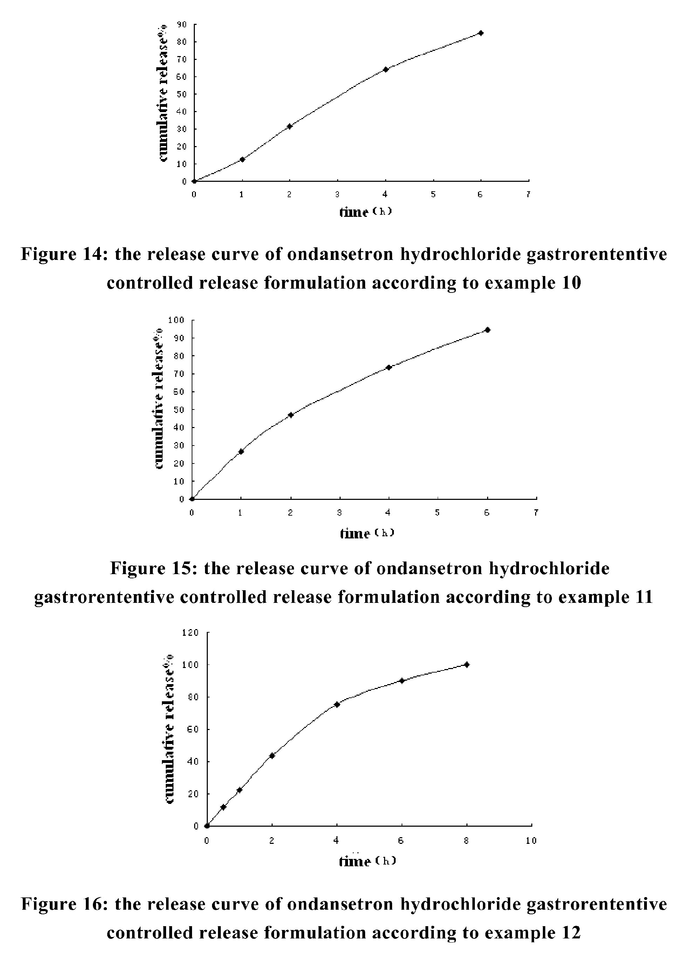

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 1, 2, 4 and 6h respectively. The cumulative release curve was shown in Figure 14. It can be seen that ondansetron gastroretentive formulation can effectively extend the residence time of drugs in the gastrointestinal tract and improve bioavailability.

### Example 11

### Gas vesicles: 1# common hard gelatin capsules (1000 capsules)

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |
| anhydrous ethanol | to 300ml |

The composition ofthe coating solution for the drug-containing layer:

| | |
|---|---|
| ondansetron hydrochloride | 1.5g |
| Eudragit RL100 | 4.5g |
| ethyl cellulose | 2.0g |
| polyethylene glycol | 1.0g |
| 90% ethanol solution | 100ml |

The composition of the coating solution for the controlled release film:

| | |
|---|---|
| Eudragit RS100 | 2.5g |
| ethyl cellulose | 2.0g |
| polyethylene glycol | 1.0g |
| triethyl citrate | 0.8g |
| 90% ethanol | 100ml |

### Preparation method:

1# capsule shells were placed in a coating pan. The temperature of tablet bed was 45°C. The coating solution for the waterproofing layer (prepared by dissolving/dispersing stearic acid, ethyl cellulose, triethyl citrate and talc powder in anhydrous ethanol) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Ondansetron hydrochloride, Eugragit RL100, ethyl cellulose and polyethylene glycol were dissolved in 90% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of ondansetron. Each capsule contained 8mg ondansetron. Eugragit RS100, ethyl cellulose, polyethylene glycol and triethyl citrate was dissolved in 90% ethanol solution to prepare the coating solution for the controlled release film. The controlled release film was coated on the outer surface of the gas vesicle using conventional film coating method. The weight gain of the coating was 20%-22%.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37°C and the rotating speed was 100r/min. The procedure was carried out according to the method and timing was started when rotation began. Samples were taken out and measured at 1, 2, 4 and 6h respectively. The cumulative release curve was shown in Figure 15. It can be seen that ondansetron gastroretentive formulation can effectively extend the residence time of drugs in the gastrointestinal tract and improve bioavailability.

### Example 12

### Gas vesicles: 3# common hard gelatin capsules (1000 capsules)

The composition of the coating solution for the waterproofing layer:

| | |
|---|---|
| ethyl cellulose | 10.0g |
| stearic acid | 15.0g |
| triethyl citrate | 5.0g |
| talc powder | 3.0g |
| 80% ethanol solution | to 300ml |

The composition of the coating solution for the drug-containing layer:

| | |
|---|---|
| ondansetron hydrochloride | 8g |
| povidone k30 | 3g |
| polyethylene glycol | 4g |
| mannitol | 12g |
| talc powder | 2.5g |
| 30% ethanol solution | to 200ml |

The composition of the coating solution for the controlled release film:

| | |
|---|---|
| ethyl cellulose | 12g |
| polyethylene glycol 4000 | 2.5g |
| HPMC | 1.5g |
| diethyl phthalate | 1.5ml |
| 80% ethanol solution | to 300ml |

### Preparation method :

3# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Ondansetron hydrochloride, mannitol, polyethylene glycol and povidone k30 were dissolved in 30% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of ondansetron. Each capsule contained 8mg ondansetron. The coating solution for the controlled-release layer was coated on the drug-containing gas vesicle using the same method to form the controlled release film. The weight gain of the coating was 10%. The resulting product was placed at 45°C for 24h to give the vesicl-type gastric floating controlled-release formulation of ondansetron hydrochloride.

### Determination of the floating-holding ability:

The floating-holding ability was determined according to the method of example 1. The results showed that the gastric floating formulation prepared in this example did not decrease within 12 hours.

### In vitro release test:

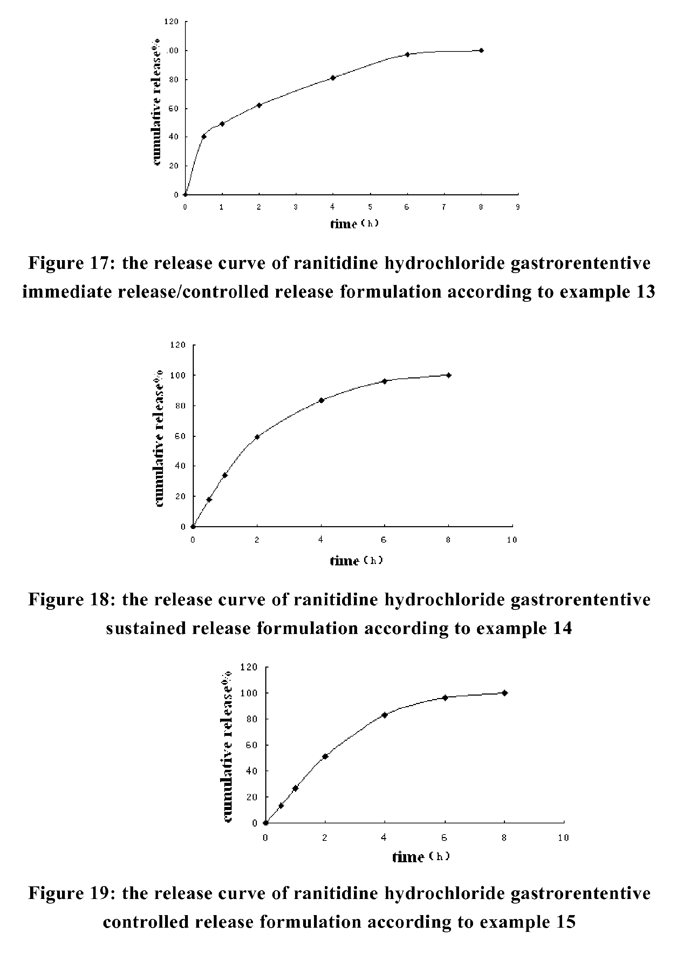

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4, 6 and 8h respectively. The cumulative release curve was shown in Figure 17.

### Example 13

### Gas vesicles: 0# common hard gelatin capsules (1000 capsules)

The composition of the coating solution for the waterproofing layer:

| | |
|---|---|
| ethyl cellulose | 12g |
| stearic acid | 20g |
| triethyl citrate | 6g |
| talc powder | 5g |
| 80% ethanol solution | to 400ml |

The composition of the coating solution for the drug-containing layer:

| | |
|---|---|
| ranitidine hydrochloride | 50g |
| povidone k30 | 40g |
| lactose | 30g |
| talc powder | 4g |
| 60% ethanol solution | to 1000ml |

The composition of the coating solution for the controlled release film:

| | |
|---|---|
| ethyl cellulose | 12g |
| polyethylene glycol 4000 | 1.5g |
| HPMC | 3.0g |
| diethyl phthalate | 3.0ml |
| 80% ethanol solution | to 400ml |

The composition of the coating solution for the immediate layer:

| | |
|---|---|
| ranitidine hydrochloride | 25 g |
| povidone k30 | 20g |
| lactose | 15g |
| talc powder | 8g |
| 30% ethanol solution | to 300ml |

### Preparation method :

0# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Ranitidine hydrochloride, lactose, talc powder and povidone k30 were dissolved/dispersed in 30% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of ranitidine. Each capsule contained 50mg ranitidine. The coating solution for the controlled-release layer prepared was coated on the drug-containing gas vesicle using the same method to form the controlled-release film. The weight gain of the coating was 12%. The resulting product was placed at 45°C for 24h and an immediate layer was coated. The weight gain of the coating was calculated based on the weight of ranitidine. The immediate layer of each capsule contained 25mg ranitidine. The vesicl-type gastric floating immediate/controlled release formulation of ranitidine hydrochloride was obtained.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 16. By preparing ranitidine into gastroretentive dual-release formulation, the residence time of the drug in the absorption site can be effectively extended and the drug release rate can be effectively controlled to maintain stable and sustained plasma concentration, thus, the frequency of administration is reduced and patient compliance is improved.

### Example 14

### Gas vesicles: 0# common hard gelatin capsules (1000 capsules)

The gas-generating agent consisted of 7.5g of citric acid and 5g of sodium bicarbonate.

The composition of the coating solution for the waterproofing layer:

| | |
|---|---|
| Eudragit RS | 15g |
| triethyl citrate | 6g |
| talc powder | 5g |
| anhydrous ethanol | to 400ml |

The composition of the coating solution for the drug-containing layer:

| | |
|---|---|
| ranitidine hydrochloride | 75 g |
| povidone k30 | 50g |
| magnesium stearate | 10g |
| 30% ethanol solution | to 400ml |

The composition of the coating solution for the sustained release film:

| | |
|---|---|
| Eudragit RS | 14g |
| Eudragit RL | 6g |
| polyethylene glycol 4000 | 1.5g |
| diethyl phthalate | 6.0ml |
| 80% ethanol solution | to 400ml |

### Preparation method :

Prescribing amount of sodium bicarbonate and citric acid were encapsuled in 0# capsule shell, locked and placed in a coating pan. The temperature of tablet bed was 45°C. The coating solution for the waterproofing layer (prepared by dissolving/dispersing Eudragit RS, triethyl citrate and talc powder in anhydrous ethanol) was sprayed into the coating pan. Ranitidine hydrochloride and magnesium stearate were suspended in 30% ethanol solution containing povidone k30 to prepare the coating solution for the drug-containing layer. The drug-containing layer was coated on the outer surface of the gas vesicle using conventional film coating method. The weight gain of the coating was calculated based on the weight of ranitidine. Each capsule contained 75mg ranitidine. The coating solution of the sustained-release layer was coated on the drug-containing gas vesicle using the same method to form the sustaineded-release film. The weight gain of the coating was 15%. The resulting product was placed at 45°C for 24h to give the vesicl-type gastric floating controlled-release formulation of ranitidine hydrochloride.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4, 6 and 8h respectively. The cumulative release curve was shown in Figure 18. By preparing ranitidine hydrochloride into gastroretentive sustained-release formulation, the residence time of the drug in the absorption site can be effectively extended and the drug release rate can be effectively controlled to maintain stable and sustained plasma concentration, thus, the frequency of administration is reduced and patient compliance is improved.

### In vivo-release test in Beagle dogs

Single dose was administered to Beagle dogs.

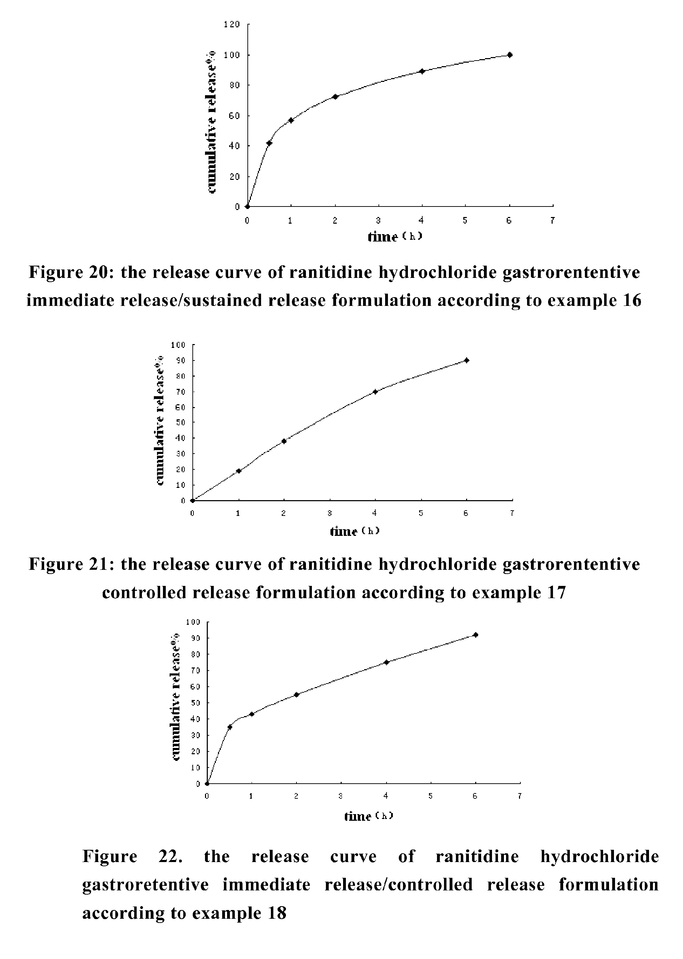

Method: 12 adult male Beagle dogs weighing 10-12kg were divided randomly into two groups (n=6). After Beagle dogs were administered uniformly with standard diet in the morning, common ranitidine hydrochloride tablets and the gastric floating sustained-release capsules of ranitidine hydrochloride prepared above were swallowed respectively. At 15min, 30min, 45min, 1h, 2h, 3h, 4h, 6h, 8h, 12h and 16h after administration, blood was collected and treated to determine plasma drug concentrations. The plasma drug concentration-time curve over time was shown in Figure 22, indicating that the plasma drug concentration of the gastric floating formulation of the present invention was more steady compared with common preparations.

### Example 15

### Gas vesicles: 0# common hard gelatin capsules (1000 capsules)

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |
| 80% ethanol solution | to 400ml |

The composition of the coating solution for the drug-containing layer:

| | |
|---|---|
| ranitidine hydrochloride | 75 g |
| povidone k30 | 40g |
| lactose | 30g |
| talc powder | 10g |
| 70% ethanol solution | to 1000ml |

The composition of the coating solution for the controlled release film:

| | |
|---|---|
| cellulose acetate | 20g |
| polyethylene glycol 4000 | 5g |
| acetone/ethanol/water (14/5/1) | to 500ml |

### Preparation method:

0# capsule shells were placed in a coating pan. The temperature of tablet bed was 45°C. The coating solution for the waterproofing layer (prepared by dissolving/dispersing ethyl cellulose, stearic acid, triethyl citrate and talc powder in 80% ethanol solution) was sprayed into the coating pan. Ranitidine hydrochloride, lactose, talc powder and povidone k30 were dissolved/dispersed in 70% ethanol solution to prepare the coating solution for the drug-containing layer. The drug-containing layer was coated on the outer surface of the gas vesicle using conventional film coating method. The weight gain of the coating was calculated based on the weight of ranitidine. Each capsule contained 75mg ranitidine. The coating solution for the controlled-release layer was coated on the drug-containing gas vesicle using the same method to form the controlled release film. The resulting product was placed at 45°C for 24h to give the final product.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 19.

### Example 16

### Gas vesicles: 0# common hard gelatin capsules (1000 capsules)

The composition of the coating solution for the waterproofing layer:

| | |
|---|---|
| ethyl cellulose | 15.0g |
| stearic acid | 10.0g |
| triethyl citrate | 4.5g |
| talc powder | 3.0g |
| 80% ethanol solution | to 400ml |

The composition of the coating solution for the drug-containing layer:

| | |
|---|---|
| ranitidine hydrochloride | 50g |
| povidone k30 | 30g |
| lactose | 30g |
| magnesium stearate | 4g |
| 70% ethanol solution | to 1000ml |

The composition of the coating solution for the sustained release film:

| | |
|---|---|
| Eudragit RS | 8g |
| Eudragit RL | 8g |
| triethyl citrate | 5 g |
| talc powder | 5g |
| 80% ethanol solution | to 300ml |

The composition of the coasting solution for the immediate release layer:

| | |
|---|---|
| ranitidine hydrochloride | 25 g |
| povidone k30 | 6g |
| talc powder | 1g |
| titanium dioxide | 1g |
| 70% ethanol solution | to 300ml |

### Preparation method :

0# capsule shells were placed in a coating pan. The temperature of tablet bed was 45°C. The coating solution for the waterproofing layer (prepared by dissolving/dispersing ethyl cellulose, stearic acid, triethyl citrate and talc powder in 80% ethanol solution) was sprayed into the coating pan. Ranitidine hydrochloride, magnesium stearate, lactose and povidone k30 were dissolved/dispersed in 70% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of ranitidine. The coating solution for the sustained-release layer was coated on the drug-containing gas vesicle using the same method to form the sustained-release film. The resulting product was placed at 45°C for 24h. The coating solution for the immediate release film was coated on the outer surface of the sustained release film using the same method to give the vesicl-type gastric floating dual-release formulation of ranitidine hydrochloride.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 20.

### Example 17

### Gas vesicles: 0# common hard gelatin capsules (1000 capsules)

The composition of the coating solution for the drug-containing layer:

| | |
|---|---|
| ranitidine hydrochloride | 75 g |
| Eudragit RS100 | 25g |
| ethyl cellulose | 15g |
| polyethylene glycol | 10g |
| 90% ethanol solution | 1000ml |

The composition of the coating solution for the controlled release film:

| | |
|---|---|
| Eudragit RS100 | 2.5g |
| ethyl cellulose | 2.0 |
| polyethylene glycol | 0.6g |
| triethyl citrate | 0.6g |
| 90% ethanol | 100ml |

### Preparation method :

Ranitidine hydrochloride, Eudragit RS100, ethyl cellulose and polyethylene glycol were dissolved in 90% ethanol solution to prepare the coating solution for the drug-containing layer. 0# intestine soluable capsule shells were placed in a coating pan. The drug-containing layer was coated on the outer surface of the gas vesicle using conventional film coating method. The weight gain of the coating was calculated based on the weight of ranitidine. Each capsule contained 40mg ranitidine. The coating solution for the controlled-release layer prepared was coated on the drug-containing gas vesicle using the same method to form the controlled-release film. The weight gain of the coating was 15% to 18%. The resulting product was placed at 45°C for 24h to give the vesicl-type gastric floating controlled release formulation of ranitidine hydrochloride.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 1, 2, 4 and 6h respectively. The cumulative release curve was shown in Figure 21.

### Example 18

### Gas vesicles: 0# common hard gelatin capsules (1000 capsules)

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |
| 80% ethanol solution | to 300ml |

The composition of the coating solution for the drug-containing layer:

| | |
|---|---|
| ranitidine hydrochloride | 50g |
| Eudragit RL100 | 45g |
| ethyl cellulose | 20g |
| polyethylene glycol | 10g |
| 90% ethanol solution | 1000ml |

The composition of the coating solution for the controlled release film:

| | |
|---|---|
| Eudragit RS100 | 10g |
| ethyl cellulose | 10g |
| polyethylene glycol | 1.0g |
| triethyl citrate | 0.8g |
| 90% ethanol | 100ml |

The composition of the coating solution for the immediate layer:

| | |
|---|---|
| ranitidine hydrochloride | 25 g |
| HPMC | 10g |
| polyethylene glycol | 4g |
| talc powder | 2g |
| 70% ethanol solution | to 200ml |

### Preparation method :

0# capsule shells were placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. Ranitidine hydrochloride, Eudragit RS100, ethyl cellulose and polyethylene glycol were dissolved in 90% ethanol solution to prepare the coating solution for the drug-containing layer. The gas vesicles prepared above were placed in the coating pan. The drug-containing layer was coated on the outer surface of the gas vesicle using conventional film coating method. The weight gain of the coating was calculated based on the weight of ranitidine. Each capsule contained 50mg ranitidine. The coating solution for the controlled-release layer was coated on the drug-containing gas vesicle using the same method to form the controlled release film. The weight gain of the coating was 22-24%. The resulting product was placed at 45°C for 24h. The coating solution for the immediate release layer was coated on the outer surface of the controlleded release film using the same method to give the vesicl-type gastric floating dual-release formulation of ranitidine hydrochloride.

### Determination of the floating-holding ability:

The floating-holding ability was determined according to the method of example 1. The results showed that the gastric floating formulation prepared in this example did not decrease within 12 hours.

### In vitro release test:

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 0.5, 1, 2, 4 and 6h respectively. The cumulative release curve was shown in Figure 22.

### Example 19

### Gas vesicles: hollow polyoxyethylene capsules (equivalent to the volume of 0# capsules, 1000 capsules)

The composition of the coating solution for the drug-containing layer:

| | |
|---|---|
| ranitidine hydrochloride | 75 g |
| PVP k30 | 75g |
| talc powder | 10g |
| 90% ethanol solution | 1000ml |

The composition of the coating solution for the controlled release film:

| | |
|---|---|
| Eudragit RS100 | 10g |
| ethyl cellulose | 10g |
| polyethylene glycol | 1.0g |
| triethyl citrate | 0.8g |
| 90% ethanol | 300ml |

### Preparation method :

Ranitidine hydrochloride, PVP k30 and talc powder were dissolved in 90% ethanol solution to prepare the coating solution for the drug-containing layer. The hollow polyoxyethylene capsules prepared were placed in the coating pan. The drug-containing layer was coated on the outer surface of the gas vesicle using conventional film coating method. The weight gain of the coating was calculated based on the weight of ranitidine. Each capsule contained 75mg ranitidine. The coating solution for the controlled-release layer prepared was coated on the drug-containing gas vesicle using the same method to form the controlled release film. The weight gain of the coating was 22-24%. The resulting product was placed at 45°C for 24h to give the gas-vesicl-type gastric floating controlled release formulation of ranitidine hydrochloride.

### Determination of the floating-holding ability:

The floating-holding ability was determined according to the method of example 1. The results showed that the gastric floating formulation prepared in this example did not decrease within 12 hours.

### In vitro release test:

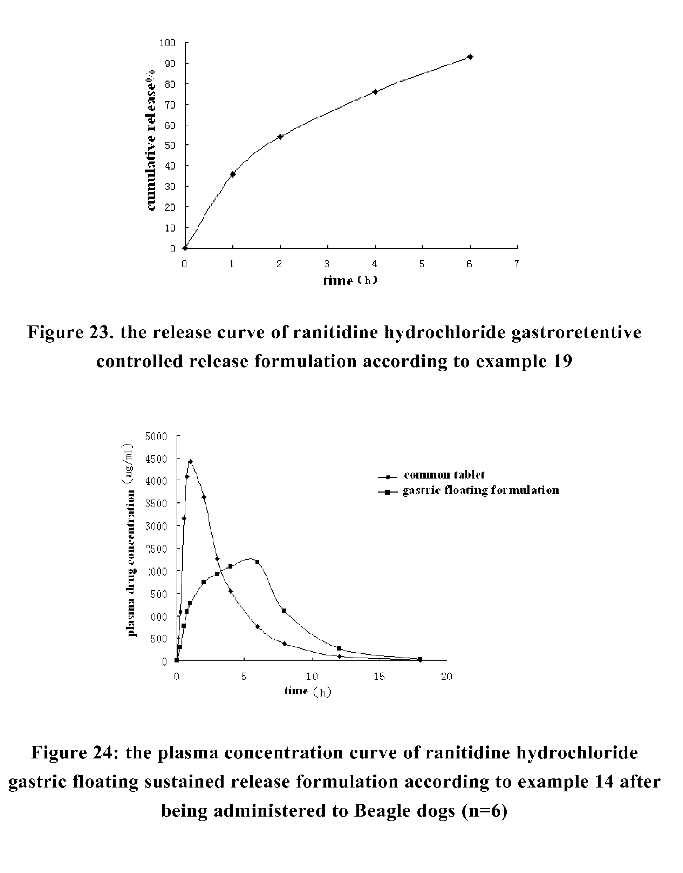

Release test was performed according to the first method for determining release performance in Chinese Pharmacopoeia 2005 edition, Part I (Appendix C). 900ml of 0.1mol/L hydrochloric acid was used as dissolution medium. The temperature was 37±0.5°C and the rotating speed was 100r/min. The procedure was carried out according to the method and timing was started when rotation began. Samples were taken out and measured at 1, 2, 4 and 6h respectively. The cumulative release curve was shown in Figure 23.

### Example 20 studies on in vivo kinetics of gastric floating

Purpose: to study the kinetics characteristics of the gas-vesicle-type gastric floating formulation.

Capsule:

| | |
|---|---|
| 0# common hard gelatin capsules | 1000 |
| barium bars | 2 bars per capsule |

The composition of the coating solution for the waterproofing layer:

| | |
|---|---|
| ethyl cellulose | 9.0g |
| stearic acid | 15.0g |
| triethyl citrate | 4.5g |
| talc powder | 3.0g |
| 80% ethanol solution | to 300ml |

The composition of the coating solution for the controlled release film:

| | |
|---|---|
| Eudragit RS100 | 2.5g |
| ethyl cellulose | 2.0g |
| polyethylene glycol | 0.8g |
| triethyl citrate | 0.8g |
| 90% ethanol | 100ml |

### Preparation method :

Each 0# capsule was encapsuled with two barium bars and not locked. The 0# capsule shell with barium bars was placed in a coating pan. The temperature of tablet bed was 45°C. The coating solution for the waterproofing layer (prepared by dissolving/dispersing stearic acid, ethyl cellulose, triethyl citrate and talc powder in 80% ethanol solution) was sprayed into the coating pan. After the weight gain of the coating reached 15%, the capsule was locked. The coating was performed again until the weight gain of the coating reached 15%. The resulting product was placed at 45°C for 12h to give a waterproofing gas vesicle. The waterproofing gas vesicles prepared above were placed in the coating pan. The controlled release film was coated on the outer surface of the waterproofing gas vesicles using conventional film coating method, to form the controlled release layer. The weight gain of the coating was 22-24%. The resulting product was placed at 45°C for 24h.

### Determination of the floating-holding ability:

The floating-holding ability was determined according to the method of example 1. The results showed that the gastric floating formulation prepared in this example did not decrease within 12 hours.

Gastric floating kinetics test was preformed using gastric floating capsules prepared above as a model drug.

### Method:

Four subjects were fasted for 12 hours. All of the subjects took meals provided for test uniformly before administration in the morning (at 8:00). After the meal, two gastric floating formulation units with two barium bars prepared above were swallowed without chewing. At 12:00 noon, meals for test were taken uniformly once again.

During the test, the subjects could drink water freely.

All subjects received X-ray detection at 1, 2, 3, 4, 6 and 8h after swallowing capsules.

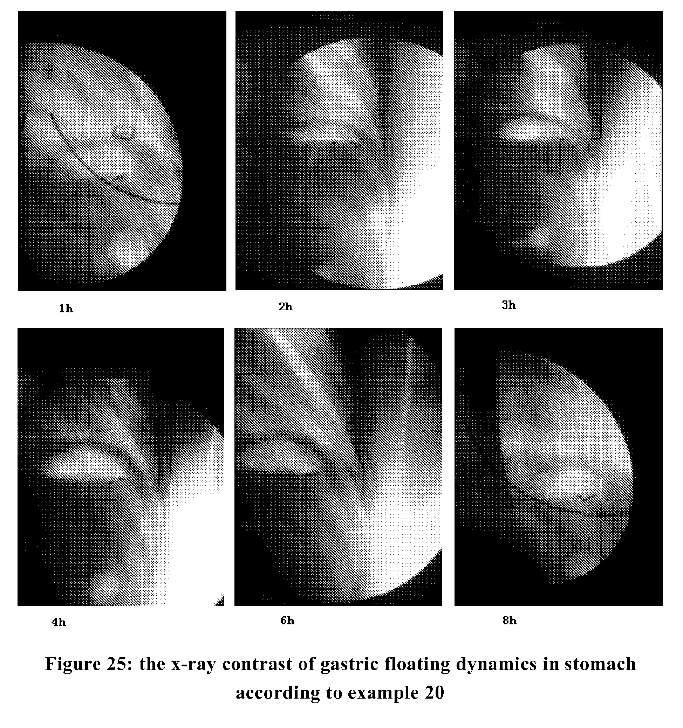

Results: the capsules still floated in the stomach at 8h after being administered to four subjects (Figure 25).

The results showed that:
(1) the residence time in the stomach of the gastric floating formulations of the above examples were much longer than that of common tablets (the residence time of common preparation is 30-120min [Wei shuli, Zhang qiang, biopharmaceutics and pharmacokinetics, 3rd edtion. Beijing: Peking University Medical Press, 1999]);
(2) the residence time in the stomach of the gastric floating formulations of the above examples were much longer than the gastric emptying time reported in literature (30-120min, seen from the above reference), and thus a true gastric retention was achieved; and/or
(3) the residence time in the stomach of the gastric floating formulations of the above examples were much longer than that of the gastroretentive formulation reported in literatures (3-5h, seen from the above reference), and thus a true gastric retention was achieved.

### Technical effects of the present invention

The gas-vesicle-type sustained/controlled release gastric floating formulations of the present invention can achieve one or more of the following technical effects:
1. The overall density is low, even lower than 0.6g/cm³;
2. The volume of the formulation can be expanded properly in vivo to get a globoid with the length of the diameter is 0.5×0.8cm to 0.7×2.0cm. Compared with microcapsules in the prior art, its size is larger, so that its effect to delay gastric emptying is significantly better and the gastric residence time is significantly longer;
3. The gastroretentive formulation of the present invention can immediately float at the initial stage and the floating-holding ability lasts and keeps constant, so the formulation can keep in a floating state for a long time;
4. The gas vesicles prepared using a capsule has a shape that is easy for patients to swallow, thereby increasing the patient's compliance;
5. The results for the stomach floating kinetics test showed that the average residence time of the gas-vesicle-type gastric floating formulation in the stomach is 5h, which is much longer than that of the common formulation reported in literatures(1.5h), therefore, it fully meets the requirements for gastric floating formulations;
6. The gastric floating formulation of the present invention can contain a gas-generating agent in the gas vesicles. After the formulation contacts with gastric juice, the gas-generating agent can produce gas. The gas expands and produces constant outward thrust, which can be effective in promoting the release of insoluble drugs, thereby avoiding drugs to be residued in the formulation resulting in a low bioavailability;
7. The gastric floating formulation of the present invention can be prepared into a sustained/controlled release formulation. As a sustained/controlled release formulation, the gastric floating formulation of the present invention can maintain stable drug release rate after being discharged from the stomach to the intestine. For drugs absorbed poorly in colon, its residence time in the absorption site is prolonged so as to administer once every 24h, so the patient's compliance is significantly improved; and/or
8. The gastric floating formulation can be prepared into a dual-release gastroretentive drug delivery system to reach the effect of administering an immediate release formulation in combination with a sustained/controlled release formulation, that is to say, the effective plasma drug concentration can be reached rapidly at the initial stage after administration, thereafter, the plasma drug concentration is maintained steady allowing the blood drug concentration to keep effective for a long time. From above, the disadvantages of the slow onset of sustained release formulations and the short duration of action of immediate release formulations can be avoided, thus, the fluctuation of the blood drug concentration is effectively reduced so as to fully guarantee the safety and effectiveness of drugs.

## Claims

1. A gastroretentive drug carrier, comprising a hollow vesicle and optional waterproofing layer and/or isolating layer coating the hollow vesicle.

2. The gastroretentive drug carrier according to claim 1, wherein the waterproofing layer is formed by a polymeric film-forming material and/or hydrophobic material, preferably, the waterproofing layer is formed by one or more materials selected from the group consisting of waxy materials, such as medicinal paraffin, cetyl alcohol, stearic acid, carnauba wax, white wax, hydrogenated castor oil; or ethyl cellulose, cellulose acetate, Eudragit S, Eudragit L, cellulose acetate phthalate (CAP), hydroxypropyl methylcellulose acetate succinate (HPMCAS), hydroxypropyl methyl cellulose phthalate (HPMCP), Opadry and the like.

3. The gastroretentive drug carrier according to claim 1, wherein the isolating layer is formed by a pharmaceutically acceptable excipient, preferably by one or more pharmaceutically acceptable excipients selected from dicalcium phosphate, starch, dextrin, sucrose, lactose, mannitol, hydroxypropyl cellulose, methyl cellulose, sodium chloride, glucose, talc powder, titanium dioxide powder, polyethylene glycol, microcrystalline cellulose, pregelatinized starch and the like, and the weight of the isolating layer accounts for 10% to 50%, more preferably 20% to 40% by weight of the hollow vesicle.

4. The gastroretentive drug carrier according to any one of claims 1-3, wherein the hollow vesicle contains a gas-generating agent, preferably, the gas-generating agent includes carbonate and/or bicarbonate and a pharmaceutically acceptable organic acid, and the amount of the gas-generating agent accounts for 1% to 20% by weight of the hollow vesicle.

5. A gastroretentive drug release system, comprising a hollow vesicle itself or the gastroretentive drug carrier according to any one of claims 1-4, and the drug-containing layer coating the hollow vesicle.

6. The gastroretentive drug release system according to claim 5, wherein the maximum radial size of the hollow vesicle is 0.5cm to 3.5cm, preferably 0.7cm to 3.0cm, more preferably 1.0cm to 2.5cm.

7. The gastroretentive drug release system according to claim 5 or 6, wherein there is a waterproofing layer coating the hollow vesicle, preferably, the waterproofing layer is formed by a polymeric film-forming material or hydrophobic material, more preferably, the waterproofing layer is formed by one or more selected from the group consisting of waxy materials, such as medicinal paraffin, cetyl alcohol, stearic acid, carnauba wax, white wax, hydrogenated castor oil; or ethyl cellulose, cellulose acetate, Eudragit S, Eudragit L, cellulose acetate phthalate (CAP), hydroxypropyl methylcellulose acetate succinate (HPMCAS), hydroxypropyl methyl cellulose phthalate (HPMCP), Opadry and the like.

8. The gastroretentive drug release system according to any one of claims 5-7, wherein there is an isolating layer consisting of a pharmaceutically acceptable excipient, which is coated on the hollow vesicle and isolates the hollow vesicle from the drug-containing layer, preferably, the weight of the isolating layer accounts for 10% to 50% by weight of the hollow vesicle, more preferably 20% to 40%.

9. The gastroretentive drug release system according to any one of claims 5-8, wherein the hollow vesicle is an empty capsule of common machine-made hard capsules, including stomach soluble capsules, intestine soluble capsules and insoluble capsules.

10. The gastroretentive drug release system according to any one of claims 5-9, wherein the hollow vesicle comprises a gas-generating agent, preferably, the gas-generating agent includes carbonate and/or bicarbonate and a pharmaceutically acceptable organic acid, and the amount of the gas-generating agent accounts for 1% to 20% by weight of the hollow vesicle.

11. The gastroretentive drug release system according to any one of claims 5-10, wherein the drug-containing layer comprises of a drug and optional pharmaceutically acceptable excipient, preferably, the drug-containing layer is a sustained or controlled release layer, and optionally, there is a immediate release drug layer coating the drug-containing layer.

12. The gastroretentive drug release system according to claim 11, wherein the controlled release layer comprises an excipient and a controlled release coating, preferably, the controlled release coating comprises a material selected from polymeric materials, plasticizers and pore-forming agents or any combination thereof.

13. The gastroretentive drug release system according to claim 11, wherein the sustained release layer comprises an excipient selected from sustained-release materials, adhesives, lubricants, penetration enhancers, solutizers or any combination thereof, and optionally comprises a gas-generating agent, preferably, the gas-generating agent is one or more selected from carbonates or bicarbonates such as sodium carbonate, sodium bicarbonate, magnesium carbonate or any combination thereof.

14. The gastroretentive drug release system according to claim 13, wherein the amount of the gas-generating agent used accounts for 1% to 20% by weight of the sustained release layer.

15. The gastroretentive drug release system according to any one of claims 5-14, wherein the drug includes one or more drugs selected from hangover medicine, drugs used to treat dementia, anesthetics, drugs used to treat acromegaly, analgesics, drugs used to treat asthma, anticancer drugs, anticoagulants, antithrombotic drugs, antiepileptic drugs, diabetes drugs, antiemetic drugs, drugs used to treat glaucoma, anti-histamine drugs, anti-infective drugs, drugs used to treat Parkinson's disease, antiplatelet drugs, anti-rheumatic drugs, anti-spasm and cholinergic drugs, antitussives, carbonic anhydrase inhibitors, cardiovascular drugs, cholinesterase inhibitors, drugs used to treat central nervous system disorders, central nervous system stimulants, contraceptives, drugs used to treat cystic fibrosis, dopamine receptor agonists, drugs used to treat endometritis, drugs used to treat erectile dysfunction, drugs used to treat infertility, gastrointestinal drugs, immuno-modulatory agents, immuno-suppressive agents, drugs used to enhance memory, migraine preparations, muscle relaxation drugs, nucleoside analogues, drugs used to treat osteoporosis, drugs used to treat parasympathomimetics, prostaglandins, psychotropic drugs, sedatives, hypnotics and tranquillizers, drugs used to treat skin diseases, steroids and hormones, as well as pharmaceutically acceptable salts or esters or prodrugs thereof; prefereably, said drug is selected from the group consisting of rosiglitazone, gentamicin, ondansetron, ranitidine, and pharmaceutically acceptable salts or esters or prodrugs thereof, or any combination thereof.

16. A gastroretentive formulation, comprising the gastroretentive drug delivery system according to any one of claims 5-15 and optional pharmaceutically acceptable excipients and/or carriers.

17. A method for preparing the gastroretentive drug release system according to any one of claims 5-15, comprising the following steps:
(1) preparing or providing a hollow vesicle, and optionally coating the hollow vesicle with a waterproofing layer and/or isolating layer to obtain the gastroretentive drug carrier according to any one of claims 1-4; and
(2) coating on the surface of the hollow vesicle or of the gastroretentive drug carrier with a drug-containing layer.

18. The method according to claim 17, wherein the drug-containing layer is a sustained or controlled release layer, and optionally, an immediate release layer is coated on the sustained or controlled release layer.

19. Use of the gastroretentive drug release system according to any one of claims 5-15 for the preparation of a formulation.

20. Use of the gastroretentive drug release system according to any one of claims 5-15 or the gastroretentive formulation according to claim 16 for preventing or treating a disease, optionally, the disease is one or more diseases selected from drunkenness, senile dementia, pain, acromegaly, asthma, cancer, cardiovascular diseases, epilepsy, diabetes, vomiting, glaucoma, allergic diseases, microbial infections, Parkinson's disease, rheumatism, convulsion, cough, endometritis, erectile dysfunction, infertility, immune deficiency and autoimmune diseases, osteoporosis, psychosis, prostatic hypertrophy and prostatitis, insomnia and skin diseases, especially diabetes, bacterial infections, cancer or gastric ulcer.

21. A method for preventing or treating a disease or condition, comprising administering the gastroretentive drug release system according to any one of claims 5-15 or the gastroretentive formulation according to claim 16 to a patient.

22. The method according to claim 21, wherein the disease or condition is one or more diseases or conditions selected from drunkenness, senile dementia, pain, acromegaly, asthma, cancer, cardiovascular diseases, epilepsy, diabetes, vomiting, glaucoma, allergic diseases, microbial infections, Parkinson's disease, rheumatism, convulsion, cough, endometritis, erectile dysfunction, infertility, immune deficiency and autoimmune diseases, osteoporosis, psychosis, prostatic hypertrophy and prostatitis, insomnia and skin diseases, especially diabetes, microorganism infections, cancer or gastric ulcer.

23. Use of the gastroretentive drug carrier according to any one of claims 1-4 for the preparation of a gastroretentive drug release system or a gastroretentive formulation.

## Amended claims

### Amended claims under Art. 19.1 PCT

**1.** A gastroretentive drug release system, comprising a hollow vesicle and the drug-containing layer coating the hollow vesicle.

**2.** The gastroretentive drug release system according to claim 1, wherein the maximum radial size of the hollow vesicle is 0.5cm to 3.5cm, preferably 0.7cm to 3.0cm, more preferably 1.0cm to 2.5cm.

**3.** The gastroretentive drug release system according to claim 1 or 2, wherein there is a waterproofing layer coating the hollow vesicle, preferably, the waterproofing layer is formed by a polymeric film-forming material or hydrophobic material, more preferably, the waterproofing layer is formed by one or more selected from the group consisting of waxy materials, such as medicinal paraffin, cetyl alcohol, stearic acid, carnauba wax, white wax, hydrogenated castor oil; or ethyl cellulose, cellulose acetate, Eudragit S, Eudragit L, cellulose acetate phthalate (CAP), hydroxypropyl methylcellulose acetate succinate (HPMCAS), hydroxypropyl methyl cellulose phthalate (HPMCP), Opadry and the like.

**4.** The gastroretentive drug release system according to any one of claims 1-3, wherein there is an isolating layer consisting of a pharmaceutically acceptable excipient, which is coated on the hollow vesicle and isolates the hollow vesicle from the drug-containing layer, wherein the isolating layer is formed by one or more pharmaceutically acceptable excipients preferably selected from calcium hydrogen phosphate, starch, dextrin, sucrose, lactose, mannitol, hydroxypropyl cellulose, methyl cellulose, sodium chloride, glucose, talc powder, titanium dioxide powder, polyethylene glycol, microcrystalline cellulose, pregelatinized starch; and preferably, the weight of the isolating layer accounts for 10% to 50% by weight of the hollow vesicle, more preferably 20% to 40%.

**5.** The gastroretentive drug release system according to any one of claims 1-4, wherein the hollow vesicle is an empty capsule of common hard capsules, including stomach soluble capsules, intestine soluble capsules and insoluble capsules.

**6.** The gastroretentive drug release system according to any one of claims 1-5, wherein the hollow vesicle comprises a gas-generating agent, preferably, the gas-generating agent includes carbonate and/or bicarbonate and a pharmaceutically acceptable organic acid, and the amount of the gas-generating agent accounts for 1% to 20% by weight of the hollow vesicle.

**7.** The gastroretentive drug release system according to any one of claims 1-6, wherein the drug-containing layer comprises of a drug and optional pharmaceutically acceptable excipient, preferably, the drug-containing layer is a sustained or controlled release layer, and optionally, there is a immediate release drug layer coating the drug-containing layer.

**8.** The gastroretentive drug release system according to claim 7, wherein the controlled release layer comprises an excipient and a controlled release coating, preferably, the controlled release coating comprises a material selected from polymeric materials, plasticizers and pore-forming agents or any combination thereof.

**9.** The gastroretentive drug release system according to claim 7, wherein the sustained release layer comprises an excipient selected from sustained-release materials, binders, lubricants, penetration enhancers, cosolvents or any combination thereof, and optionally comprises a gas-generating agent, preferably, the gas-generating agent is one or more selected from carbonates or bicarbonates such as sodium carbonate, sodium bicarbonate, magnesium carbonate or any combination thereof.

**10.** The gastroretentive drug release system according to claim 9, wherein the amount of the gas-generating agent used accounts for 1% to 20% by weight of the capsule.

**11.** The gastroretentive drug release system according to any one of claims 1-10, wherein the drug includes one or more drugs selected from hangover medicine, drugs used to treat dementia, anesthetics, drugs used to treat acromegaly, analgesics, drugs used to treat asthma, anticancer drugs, anticoagulants, antithrombotic drugs, antiepileptic drugs, diabetes drugs, antiemetic drugs, drugs used to treat glaucoma, anti-histamine drugs, anti-infective drugs, drugs used to treat Parkinson's disease, antiplatelet drugs, anti-rheumatic drugs, anti-spasm and cholinergic drugs, antitussives, carbonic anhydrase inhibitors, cardiovascular drugs, cholinesterase inhibitors, drugs used to treat central nervous system disorders, central nervous system stimulants, contraceptives, drugs used to treat cystic fibrosis, dopamine receptor agonists, drugs used to treat endometritis, drugs used to treat erectile dysfunction, drugs used to treat infertility, gastrointestinal drugs, immuno-modulatory agents, immuno-suppressive agents, drugs used to enhance memory, migraine preparations, muscle relaxation drugs, nucleoside analogues, drugs used to treat osteoporosis, drugs used to treat parasympathomimetics, prostaglandins, psychotropic drugs, sedatives, hypnotics and tranquillizers, drugs used to treat skin diseases, steroids and hormones, as well as pharmaceutically acceptable salts or esters or prodrugs thereof; prefereably, said drug is selected from the group consisting of rosiglitazone, gentamicin, ondansetron, ranitidine, and pharmaceutically acceptable salts or esters or prodrugs thereof, or any combination thereof.

**12.** A gastroretentive formulation, comprising the gastroretentive drug release system according to any one of claims 1-11 and optional pharmaceutically acceptable excipients and/or carriers.

**13.** A method for preparing the gastroretentive drug release system according to any one of claims 1-11, comprising the following steps:
(1) preparing or providing a hollow vesicle; and
(2) coating on the surface of the hollow vesicle with a drug-containing layer.

**14.** The method according to claim 13, wherein the drug-containing layer is a sustained or controlled release layer, and optionally, an immediate release layer is coated on the sustained or controlled release layer.

**15.** Use of the gastroretentive drug release system according to any one of claims 1-11 for the preparation of a formulation.

**16.** Use of the gastroretentive drug release system according to any one of claims 1-11 or the gastroretentive formulation according to claim 12 for preventing or treating a disease, optionally, the disease is one or more diseases selected from drunkenness, senile dementia, pain, acromegaly, asthma, cancer, cardiovascular diseases, epilepsy, diabetes, vomiting, glaucoma, allergic diseases, microbial infections, Parkinson's disease, rheumatism, convulsion, cough, endometritis, erectile dysfunction, infertility, immune deficiency and autoimmune diseases, osteoporosis, psychosis, prostatic hypertrophy and prostatitis, insomnia and skin diseases, especially diabetes, bacterial infections, cancer or gastric ulcer.

**17.** A method for preventing or treating a disease or condition, comprising administering the gastroretentive drug release system according to any one of claims 1-11 or the gastroretentive formulation according to claim 12 to a patient.

**18.** The method according to claim 17, wherein the disease or condition is one or more diseases or conditions selected from drunkenness, senile dementia, pain, acromegaly, asthma, cancer, cardiovascular diseases, epilepsy, diabetes, vomiting, glaucoma, allergic diseases, microbial infections, Parkinson's disease, rheumatism, convulsion, cough, endometritis, erectile dysfunction, infertility, immune deficiency and autoimmune diseases, osteoporosis, psychosis, prostatic hypertrophy and prostatitis, insomnia and skin diseases, especially diabetes, microorganism infections, cancer or gastric ulcer.
